# EUROPEAN PATENT APPLICATION

(11) **EP 4 043 034 A1**
(43) Date of publication of application: **17.08.2022**
(21) Application number: 20874161.1
(22) Date of filing: 09.10.2020
(51) Int. Cl.: A61K 39/395, A61K 47/54, A61K 47/64, A61P 35/00

(54) **LYSOSOME-TARGETING ANTIBODY-DRUG CONJUGATE AND APPLICATION THEREOF**

(30) Priority: 09.10.2019 CN 201910953086
(71) Applicant: Sun Yat-Sen University, Guangzhou, Guangdong 510275 (CN)
(72) Inventor: CAI, Xiaoqing, Guangzhou Guangdong 510006 (CN); JIANG, Xianxing, Guangzhou Guangdong 510006 (CN)
(74) Representative: SSM Sandmair
(86) International application number: PCT/CN2020/119996
(87) International publication number: WO 2021/068890

(57) **Abstract**

Disclosed in the present invention is a lysosome-targeting antibody-drug conjugate and use thereof. The structure of the antibody-drug conjugate is Drₙ₁AbOₙ₂; wherein, Dr is a drug, Ab is an antibody, and O is a lysosome-targeting small molecule or a functional peptide for increasing the lysosomal targeting ability of the antibody-drug conjugate; n1 and n2 are integers greater than or equal to 1, and n1 and n2 are identical or different.

## Description

### Field of the Invention

The present invention belongs to the technical field of biomedical technology, and relates to an antibody-drug conjugate and use thereof, in particular to a lysosome-targeting antibody-drug conjugate and use thereof.

### Background of the Invention

Antibody-drug conjugates (ADCs) have become a very promising therapeutic approach in anti-cancer therapy that combines the target specificity of antibodies and cytotoxicity of chemotherapeutic small-molecule drugs. The mechanism of action of ADCs requires the specific recognition and binding of antibodies to antigens on cancer cell surfaces, followed by endocytosis of ADCs in an antigen-mediated manner. Upon internalization into cancer cells, ideal ADCs will go through the endosome-lysosome intracellular pathway to release the cytotoxic small-molecule drug, which will then reach its target (commonly tubulin or DNA) to exert its cytotoxicity and induce cell death.

ADCs are internalized into eukaryotic cells mainly through three distinct pathways: (1) clathrin-mediated endocytosis, (2) caveolin-mediated endocytosis, and (3) macropinocytosis. Among these three pathways, both clathrin-mediated endocytosis and caveolin-mediated endocytosis are antigen-dependent, with clathrin-mediated endocytosis being the only one that can be efficiently processed through the lysosomal system. Antibodies internalized through caveolin-mediated endocytosis tend to accumulate in the endoplasmic reticulum or Golgi apparatus. ADCs that enter cells through non-selective macropinocytosis may either be fused with lysosomes through macropinosomes or be removed outside the cell in an efflux step, which mainly relies on the cell types. For example, macropinosomes in human epidermal carcinoma cell A431 cells are more likely to be excreted from cells instead of fusing with lysosomes. In addition, the Fc segment of full-length antibody can interact with the neonatal Fc receptor (FcRn) in a pH-dependent manner, thus promoting the recycling of antibody from normal lysosomal degradation pathway to the cell surface and prolonging the half-life of the antibody.

Whether ADCs can be effectively internalized and fused into lysosomes is crucial for their therapeutic efficacy. Only when ADCs are degraded in lysosomes to release small-molecule drugs, which can exert their cytotoxicity through interacting with their targets (commonly tubulin or DNA), ADCs can ultimately elicit their anti-tumor activity. Studies have shown that antigens on the surface of cancer cells are in a dynamic process of trafficking in and out of plasma membranes, with some of ADCs being recycled backed to the surface of plasma membranes. Therefore, the number of drug molecules released in the cytoplasm is very limited; especially for cancer cells with very low antigen expression, the number of cytotoxic molecules that reach the cytoplasm is even less. Therefore, most existing ADCs are incapable to kill cancer cells effectively, and thus failing to achieve the ideal therapeutic effect. In addition, the recent research results showed that the diminution in accumulation or proteolysis of ADCs in lysosomes could be responsible for drug resistance to ADCs in clinics.

Therefore, it is necessary to provide a new approach to solve the problems of ADCs existing in the prior art.

### Summary of the Invention

The purpose of the present invention is to solve the problems existing in the prior art, and to provide a lysosome-targeting antibody-drug conjugate and its use. The technique provides a simple and versatile method for directing the antibody-drug conjugate into lysosomes for effective release of cytotoxic small-molecules, through modifying the antibody component of the antibody-drug conjugate with a lysosome-targeting small molecule or a functional peptide. The technique can improve the lysosomal trafficking, enhance cellular uptake, and increase internalization rate, and therefore represents a unique and efficient strategy to improve the therapeutic effect of ADCs.

To achieve the above purpose, the present invention provides a lysosome-targeting antibody-drug conjugate, and the structure of the antibody-drug conjugate is as follows:
Drₙ₁AbOₙ₂;
Wherein, Dr is a drug;
Ab is an antibody;
O is a lysosome-targeting small molecule or a functional peptide for increasing the lysosome-targeting ability of the antibody-drug conjugate;
n1 and n2 are integers greater than or equal to 1, and n1 and n2 are identical or different.

Preferably, the antibody is a monoclonal antibody or a nanobody, and the monoclonal antibody is one selected from Glembatumumab, Vandortuzumab, Tisotumab, Enfortumab, Cetuximab, Coltuximab, Lorvotuzumab, Gemtuzumab, Trastuzumab, and Ladiratuzumab; the nanobody is one selected from 7D12, EGA1, 9G8, C7b, 5F7, and 2Rs15d.

Preferably, the functional peptide is a lysosome-sorting peptide, a cell-penetrating peptide, or a combination of the lysosome-sorting peptide and the cell-penetrating peptide; the functional peptide is conjugated to the C-terminal of the antibody.

Preferably, the lysosome-sorting peptide is one selected from NPXY, YXXØ, [DE]XXXL[LI], DXXLL, NPFXD, and NPFXXD, wherein the X is any amino acid, and the Ø is any amino acid with a bulky hydrophobic side chain, the N is asparagine, the P is proline, the Y is tyrosine, the E is glutamic acid, the L is leucine, the I is isoleucine, the F is phenylalanine, and the D is aspartic acid, the [DE] is represented as one of the D or the E, and the [LI] is represented as one of the L or the I.

Preferably, the cell-penetrating peptide is one selected from a cationic cell-penetrating peptide, an amphiphilic cell-penetrating peptide, and a hydrophobic cell-penetrating peptide.

Preferably, the cationic penetrating peptide is one selected from polyarginine, HIV-TAT, DPV1047, and Penetratin; the amphiphilic penetrating peptide is one selected from MPG, PVEC, MAP, Pept-1, Transportan, and P28; the hydrophobic penetrating peptide is one selected from C105Y, PFVYLI, and Pep-1.

Preferably, the lysosome-targeting small molecule is conjugated to aside chain of an amino acid of the antibody.

Preferably, the side chain of the amino acid is a side chain of a natural amino acid or a bioorthogonal group.

Preferably, the side chain of the natural amino acid is one selected from a side chain of lysine and a side chain of cysteine; the bioorthogonal group is one selected from an azide group, an alkynyl group, an aldehyde group, a ketone group, and a fluorosulfate group.

Preferably, the lysosome-targeting small molecule is a pH-sensitive group-containing compound or a sugar-group containing compound.

Preferably, the pH-sensitive group is one selected from sulfonic acid group, 4-morpholinyl group, 2-(2-morpholinoethylamino)ethyl group, and polyethylene glycol group.

Preferably, the sugar-group is one selected from glucose, mannose, mannose-6-phosphate, and galactose.

The present invention also provides a use of the above-mentioned lysosome-targeting antibody-drug conjugate in an anticancer drug.

Compared with the prior art, the lysosome-targeting antibody-drug conjugate of the present invention has the following advantages:
The lysosome-targeting antibody-drug conjugate of the present invention has the antibody component modified by a lysosome-targeting small molecule or a functional peptide, so that the antibody-drug conjugate exhibits significantly stronger cellular internalization capacity, faster internalization rate, and more efficient lysosomal trafficking activity. Compared with the unmodified antibody-drug conjugate, the lysosome-targeting antibody-drug conjugate of the present invention possess higher lysosome-targeting property, and show stronger ability to inhibit cancer cell proliferation with higher cancer cell cytotoxicity and improved in vitro antitumor potency; thus providing a new possibility for improving the therapeutic effects of ADCs.

### Description of the Drawings

In order to make the content of the present invention easier to understand, the present invention will be described in further details below according to specific embodiments of the present invention and in conjunction with the accompanying drawings, wherein:
Fig. 1 is the MALDI-TOF mass spectrum of 7D12-TAMRA-LPETG-His₆ described in Example 1 of the present invention;
Fig. 2 is the MALDI-TOF mass spectrum of 7D12-MMAF-LPETG-His₆ described in Example 1 of the present invention;
Fig. 3 is the sequences of the functional peptides and the molecular structures of the lysosome-targeting small molecules described in Examples 2 and 3 of the present invention, compound 1 is sulfomethanoic acid, compound 2 is 2-morpholinoacetic acid, and compound 3 is 3-((2-morpholin-4-ylethyl)amino)propanoic acid, compound 4 is 2-(2-(2-methoxyethoxy)ethoxy)acetic acid, and compound 5 is glucose-PEG2-triazole-PEG-acetic acid;
Fig. 4a is sodium dodecyl sulfate-polyacrylamide gel electrophoresis (SDS-PAGE) and anti-His₆-tag western blot characterizations of the 7D12-peptide conjugates described in Example 2 of the present invention;
Fig. 4b is sodium dodecyl sulfate-polyacrylamide gel electrophoresis (SDS-PAGE) characterization of Trastuzumab-peptide conjugate;
Fig. 5 is the MALDI-TOF mass spectrum of 7D12-TAMRA-SA described in Example 3 of the present invention;
Fig. 6 is the MALDI-TOF mass spectrum of 7D12-TAMRA-PEG3 described in Example 3 of the present invention;
Fig. 7 is the MALDI-TOF mass spectrum of 7D12-TAMRA-morpholine described in Example 3 of the present invention;
Fig. 8 is the MALDI-TOF mass spectrum of 7D12-TAMRA-EtNH-morpholine described in Example 3 of the present invention;
Fig. 9 is the MALDI-TOF mass spectrum of 7D12-TAMRA-glucose described in Example 3 of the present invention;
Fig. 10 is the MALDI-TOF mass spectrum of 7D12-MMAF-SA described in Example 3 of the present invention;
Fig. 11 is the MALDI-TOF mass spectrum of 7D12-MMAF-PEG3 described in Example 3 of the present invention;
Fig. 12 is the MALDI-TOF mass spectrum of the 7D12-MMAF-morpholine described in Example 3 of the present invention;
Fig. 13 is the MALDI-TOF mass spectrum of 7D12-MMAF-EtNH-morpholine described in Example 3 of the present invention;
Fig. 14 is the MALDI-TOF mass spectrum of 7D12-MMAF-glucose described in Example 3 of the present invention;
Fig. 15 is the characterization results of the internalization properties of the 7D12 conjugates described in Example 4 of the present invention;
Fig. 16 is the time course of the internalization of 7D12 conjugates described in Example 4 of the present invention in A431 cells;
Fig. 17a is the confocal laser scanning microscope images of the 7D12-TAMRA, 7D12-TAMRA-LSP, 7D12-TAMRA-R₉, 7D12-TAMRA-R₉-LSP, 7D12-TAMRA-TAT, and 7D12-TAMRA-TAT-LSP described in Example 5 of the present invention;
Fig. 17b is the confocal laser scanning microscope images of 7D12-TAMRA, 7D12-TAMRA-SA, 7D12-TAMRA-PEG3, 7D12-TAMRA-morpholine, 7D12-TAMRA-EtNH-morpholine, and 7D12-TAMRA-glucose described in Example 5 of the present invention;
Fig. 17c is the confocal laser scanning microscope images of Trastuzumab-TAMRA, Trastuzumab-TAMRA-R9, Trastuzumab-TAMRA-R9-LSP, and Trastuzumab-TAMRA-LSP described in Example 5 of the present invention;
Fig. 18a is the Pearson's correlation coefficients between the conjugate and lysosomes shown in Fig. 17a;
Fig. 18b is the Pearson's correlation coefficients between the conjugate and lysosomes shown in Fig. 17b;
Fig. 18c is the Pearson's correlation coefficients between the conjugate and lysosomes shown in Fig. 17c;
Fig. 19a is the calibration curve plotted between the concentration of TAMRA-labeled antibody and the corresponding fluorescence intensity;
Fig. 19b is the quantifications of antibody internalized into cells;
Fig. 20 is the effect of temperature and endocytosis inhibitors on the internalization of 7D12 conjugates;
Fig. 21 is the co-localization of 7D12-TAMRA-LSP, 7D12-TAMRA-TAT and 7D12-TAMRA-R₉ with clathrin or caveolin-1;
Fig. 22 is the Pearson's correlation coefficients between 7D12-TAMRA-LSP, 7D12-TAMRA-TAT and 7D12-TAMRA-R₉ with clathrin or caveolin-1;
Fig. 23 is the cell viability versus the concentration of MMAF, 7D12 or the conjugates tested;
Fig. 24 is the confocal laser scanning microscope images of three-dimensional tumor spheroids;
Fig. 25 is the inhibitory effect on the growth of tumor spheroids at varying concentrations of 7D12 conjugates;
Fig. 26 is the representative images of tumor spheroids;
Fig. 27 is the schematic diagram of the mechanism of action of the antibody-drug conjugates of the present invention.

### Detailed Description of the Invention

The embodiments of the present invention will be described in detail below with reference to the examples, and should be understandable to the professionals in the field. The following examples are only used to illustrate the present invention, and should not be considered to limit the scope of the present invention. If no specific conditions are indicated in the examples, the conventional conditions or the conditions suggested by the manufacturer are applied. The reagents or instruments used without the manufacturer's indication are conventional products available from the market.

### Example 1

### Preparation of Antibody-Drug Conjugates

### (1) Choosing an antibody

The antibody of the present invention is a monoclonal antibody or a nanobody, and the monoclonal antibody is one selected from Glembatumumab, Vandortuzumab, Tisotumab, Enfortumab, Cetuximab, Coltuximab, Lorvotuzumab, Gemtuzumab, Trastuzumab, and Ladiratuzumab; the nanobody is one selected from 7D12, EGA1, 9G8, C7b, 5F7, and 2Rs15d. The amino acid sequences of the above antibodies are shown below:

### ① Nanobody

The amino acid sequence of 7D12 is:

QVKLEESGGGSVQTGGSLRLTCAASGRTSRSYGMGWFRQAPGKEREFVS
GISWRGDSTGYADSVKGRFTISRDNAKNTVDLQMNSLKPEDTAIYYCAA
AAGSAWYGTLYEYDYWGQGTQVTVSS.

The amino acid sequence of EGA1 is:

QVQLQESGGGLVQPGGSLRLSCAASGRTFSSYAMGWFRQAPGKQREFVAA
IRWSGGYTYYTDSVKGRFTISRDNAKTTVYLQMNSLKPEDTAVYYCAATY
LSSDYSRYALPQRPLDYDYWGQGTQVTVSS.

The amino acid sequence of 9G8 is:

EVQLVESGGGLVQAGGSLRLSCAASGRTFSSYAMGWFRQAPGKEREFVVA
INWSSGSTYYADSVKGRFTISRDNAKNTMYLQMNSLKPEDTAVYYCAAGY
QINSGNYNFKDYEYDYWGQGTQVTVSSA.

The amino acid sequence of C7b is:

QVQLVQSGGGLVQAGGSLRLSCAASGRTFSSYAMAWFRQAPGKEREFVAA
ISWSGANIYVADSVKGRFTISRDNAKDTVYLQMNSLKPEDTAVYYCAVKL
GFAPVEERQYDYWGQGTQVTVSS.

The amino acid sequence of 5F7 is:

EVQLVESGGGLVQAGGSLRLSCAASGITFSINTMGWYRQAPGKQRELVALI
SSIGDTYYADSVKGRFTISRDNAKNTVYLQMNSLKPEDTAVYYCKRFRTAA
QGTDYWGQGTQVTVSS.

The amino acid sequence of 2Rs15d is:

QVQLQESGGGSVQAGGSLKLTCAASGYIFNSCGMGWYRQSPGRERELVS
RISGDGDTWHKESVKGRFTISQDNVKKTLYLQMNSLKPEDTAVYFCAVCY
NLETYWGQGTQVTVSS.

### ② Monoclonal antibody

The amino acid sequence of Glembatumumab is:
H-GAMMA1 : 
QVQLQESGPGLVKPSQTLSLTCTVSGGSISSFNYYWSWIRHHPGKGLEWI
GYIYYSGSTYSNPSLKSRVTISVDTSKNQFSLTLSSVTAADTAVYYCARGY
NWNYFDYWGQGTLVTVSSASTKGPSVFPLAPCSRSTSESTAALGCLVKDY
FPEPVTVSWNSGALTSGVHTFPAVLQSSGLYSLSSVVTVPSSNFGTQTYTC
NVDHKPSNTKVDKTVERKCCVECPPCPAPPVAGPSVFLFPPKPKDTLMISR
TPEVTCVVVDVSHEDPEVQFNWYVDGVEVHNAKTKPREEQFNSTFRVVS
VLTVVHQDWLNGKEYKCKVSNKGLPAPIEKTISKTKGQPREPQVYTLPPS
REEMTKNQVSLTCLVKGFYPSDIAVEWESNGQPENNYKTTPPMLDSDGSF
FLYSKLTVDKSRWQQGNVFSCSVMHEALHNHYTQKSLSLSPGK.
L-KAPPA:

EIVMTQSPATLSVSPGERATLSCRASQSVDNNLVWYQQKPGQAPRLLIYG
ASTRATGIPARFSGSGSGTEFTLTISSLQSEDFAVYYCQQYNNWPPWTFGQ
GTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQ
GLSSPVTKSFNRGEC.

The amino acid sequence of Vandortuzumab is:
H-GAMMA1 : 
EVQLVESGGGLVQPGGSLRLSCAVSGYSITSDYAWNWVRQAPGKGLEWV
GYISNSGSTSYNPSLKSRFTISRDTSKNTLYLQMNSLRAEDTAVYYCARER
NYDYDDYYYAMDYWGQGTLVTVSSASTKGPSVFPLAPSSKSTSGGTAAL
 
GCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSL
SPGK.
L-KAPPA:

DIQMTQSPSSLSASVGDRVTITCKSSQSLLYRSNQKNYLAWYQQKPGKAP
KLLIYWASTRESGVPSRFSGSGSGTDFTLTISSLQPEDFATYYCQQYYNYPR
TFGQGTKVEIKRTVAAPSVFIFPPSDEQLKSGTASVVCLLNNFYPREAKVQ
WKVDNALQSGNSQESVTEQDSKDSTYSLSSTLTLSKADYEKHKVYACEV
THQGLSSPVTKSFNRGEC.

The amino acid sequence of Tisotumab is:
H-GAMMA1 : 
EVQLLESGGGLVQPGGSLRLSCAASGFTFSNYAMSWVRQAPGKGLEWVS
SISGSGDYTYYTDSVKGRFTISRDNSKNTLYLQMNSLRAEDTAVYYCARS
PWGYYLDSWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYIC
NVNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK.
L-KAPPA:

DIQMTQSPPSLSASAGDRVTITCRASQGISSRLAWYQQKPEKAPKSLIYAA
SSLQSGVPSRFSGSGSGTDFTLTISSLQPEDFATYYCQQYNSYPYTFGQGTK
LEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSP
VTKSFNRGEC.

The amino acid sequence of Enfortumab is:
H-GAMMA1 : 
EVQLVESGGGLVQPGGSLRLSCAASGFTFSSYNMNWVRQAPGKGLEWVS
YISSSSSTIYYADSVKGRFTISRDNAKNSLSLQMNSLRDEDTAVYYCARAY
YYGMDVWGQGTTVTVSSASTKGPSVFPLAPSSKSTSGGTAALGCLVKDY
FPEPVTVSWNSGALTSGVHTFPAVLQSSGLYSLSSVVTVPSSSLGTQTYICN
VNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDS
DGSFFLYSKLTVDKSRWQQGNVFSCSVMHEALHNHYTQKSLSLSPGK.
L-KAPPA:

DIQMTQSPSSVSASVGDRVTITCRASQGISGWLAWYQQKPGKAPKFLIYA
ASTLQSGVPSRFSGSGSGTDFTLTISSLQPEDFATYYCQQANSFPPTFGGGT
KVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLS
SPVTKSFNRGEC.

The amino acid sequence of Cetuximab is:
H-GAMMA1 : 
QVQLKQSGPGLVQPSQSLSITCTVSGFSLTNYGVHWVRQSPGKGLEWLG
VIWSGGNTDYNTPFTSRLSINKDNSKSQVFFKMNSLQSNDT
AIYYCARALTYYDYEFAYWGQGTLVTVSAASTKGPSVFPLAPSSKSTSGG
TAALGCLVKDYFPEPVTVSWNSGALTSGVHTFPAVLQSSG
LYSLSSVVTVPSSSLGTQTYICNVNHKPSNTKVDKRVEPKSCDKTHTCPPC
PAPELLGGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVYTLPPSREEM
TKNQVSLTCLVKGFYPSDIAVEWESNGQPENNYKTTPPVLDSDGSFFLYSK
LTVDKSRWQQGNVFSCSVMHEALHNHYTQKSLSLSPGK.
L-KAPPA:

DILLTQSPVILSVSPGERVSFSCRASQSIGTNIHWYQQRTNGSPRLLIKYASE
SISGIPSRFSGSGSGTDFTLSINSVESEDIADYYCQQ
NNNWPTTFGAGTKLELKRTVAAPSVFIFPPSDEQLKSGTASVVCLLNNFYP
REAKVQWKVDNALQSGNSQESVTEQDSKDSTYSLSSTLT
LSKADYEKHKVYACEVTHQGLSSPVTKSFNRGEC.

The amino acid sequence of Coltuximab is:
H-GAMMA1 : 
QVQLVQPGAEVVKPGASVKLSCKTSGYTFTSNWMHWVKQAPGQGLEWI
GEIDPSDSYTNYNQNFQGKAKLTVDKSTSTAYMEVSSLRSDD
TAVYYCARGSNPYYYAMDYWGQGTSVTVSSASTKGPSVFPLAPSSKSTS
GGTAALGCLVKDYFPEPVTVSWNSGALTSGVHTFPAVLQSS
GLYSLSSVVTVPSSSLGTQTYICNVNHKPSNTKVDKKVEPKSCDKTHTCP
PCPAPELLGGPSVFLFPPKPKDTLMISRTPEVTCVVVDVS
HEDPEVKFNWYVDGVEVHNAKTKPREEQYNSTYRVVSVLTVLHQDWLN
GKEYKCKVSNKALPAPIEKTISKAKGQPREPQVYTLPPSRDE
LTKNQVSLTCLVKGFYPSDIAVEWESNGQPENNYKTTPPVLDSDGSFFLYS
KLTVDKSRWQQGNVFSCSVMHEALHNHYTQKSLSLSPGK.
L-KAPPA:

EIVLTQSPAIMSASPGERVTMTCSASSGVNYMHWYQQKPGTSPRRWIYDT
SKLASGVPARFSGSGSGTDYSLTISSMEPEDAATYYCHQR
GSYTFGGGTKLEIKRTVAAPSVFIFPPSDEQLKSGTASVVCLLNNFYPREA
KVQWKVDNALQSGNSQESVTEQDSKDSTYSLSSTLTLSK
ADYEKHKVYACEVTHQGLSSPVTKSFNRGEC.

The amino acid sequence of Lorvotuzumab is:
H-GAMMA1 : 
QVQLVESGGGVVQPGRSLRLSCAASGFTFSSFGMHWVRQAPGKGLEWVA
YISSGSFTIYYADSVKGRFTISRDNSKNTLYLQMNSLRAED
TAVYYCARMRKGYAMDYWGQGTLVTVSSASTKGPSVFPLAPSSKSTSGG
TAALGCLVKDYFPEPVTVSWNSGALTSGVHTFPAVLQSSGL
YSLSSVVTVPSSSLGTQTYICNVNHKPSNTKVDKKVEPKSCDKTHTCPPCP
 
APELLGGPSVFLFPPKPKDTLMISRTPEVTCVVVDVSHE
DPEVKFNWYVDGVEVHNAKTKPREEQYNSTYRVVSVLTVLHQDWLNGK
EYKCKVSNKALPAPIEKTISKAKGQPREPQVYTLPPSRDELT
KNQVSLTCLVKGFYPSDIAVEWESNGQPENNYKTTPPVLDSDGSFFLYSKL
TVDKSRWQQGNVFSCSVMHEALHNHYTQKSLSLSPGK.
L-KAPPA:

DVVMTQSPLSLPVTLGQPASISCRSSQIIIHSDGNTYLEWFQQRPGQSPRRL
IYKVSNRFSGVPDRFSGSGSGTDFTLKISRVEAEDVGV
YYCFQGSHVPHTFGQGTKVEIKRTVAAPSVFIFPPSDEQLKSGTASVVCLL
NNFYPREAKVQWKVDNALQSGNSQESVTEQDSKDSTYSL
SSTLTLSKADYEKHKVYACEVTHQGLSSPVTKSFNRGEC.

The amino acid sequence of Gemtuzumab is:
H-GAMMA1 : 
EVQLVQSGAEVKKPGSSVKVSCKASGYTITDSNIHWVRQAPGQSLEWIG
YIYPYNGGTDYNQKFKNRATLTVDNPTNTAYMELSSLRSED
TAFYYCVNGNPWLAYWGQGTLVTVSSASTKGPSVFPLAPCSRSTSESTAA
LGCLVKDYFPEPVTVSWNSGALTSGVHTFPAVLQSSGLYS
LSSVVTVPSSSLGTKTYTCNVDHKPSNTKVDKRVESKYGPPCPPCPAPEFL
GGPSVFLFPPKPKDTLMISRTPEVTCVVVDVSQEDPEVQ
FNWYVDGVEVHNAKTKPREEQFNSTYRVVSVLTVLHQDWLNGKEYKCK
VSNKGLPSSIEKTISKAKGQPREPQVYTLPPSQEEMTKNQVS
LTCLVKGFYPSDIAVEWESNGQPENNYKTTPPVLDSDGSFFLYSRLTVDKS
RWQEGNVFSCSVMHEALHNHYTQKSLSLSLGK.
L-KAPPA:

DIQLTQSPSTLSASVGDRVTITCRASESLDNYGIRFLTWFQQKPGKAPKLL
MYAASNQGSGVPSRFSGSGSGTEFTLTISSLQPDDFATY
YCQQTKEVPWSFGQGTKVEVKRTVAAPSVFIFPPSDEQLKSGTASVVCLL
NNFYPREAKVQWKVDNALQSGNSQESVTEQDSKDSTYSLS
STLTLSKADYEKHKVYACEVTHQGLSSPVTKSFNRGEC.

The amino acid sequence of Trastuzumab is:
H-GAMMA1 : 
EVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRW
GGDGFYAMDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQ
TYICNVNHKPSNTKVDKKVEPKSCDKTHTCPPCPAPELLGGPSVFLFPPKP
KDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSP
G.
L-KAPPA:

DIQMTQSPSSLSASVGDRVTITCRASQDVNTAVAWYQQKPGKAPKLLIYS
ASFLYSGVPSRFSGSRSGTDFTLTISSLQPEDFATYYCQQHYTTPPTFGQGT
KVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLS
SPVTKSFNRGEC.

The amino acid sequence of Ladiratuzumab is:
H-GAMMA1 : 
QVQLVQSGAEVKKPGASVKVSCKASGLTIEDYYMHWVRQAPGQGLEW
MGWIDPENGDTEYGPKFQGRVTMTRDTSINTAYMELSRLRSDDTAVYYC
AVHNAHYGTWFAYWGQGTLVTVSSASTKGPSVFPLAPSSKSTSGGTAAL
GCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFP
PKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLS
PGK.
L-KAPPA:

DVVMTQSPLSLPVTLGQPASISCRSSQSLLHSSGNTYLEWYQQRPGQSPRP
LIYKISTRFSGVPDRFSGSGSGTDFTLKISRVEAEDVGVYYCFQGSHVPYTF
GGGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVT
HQGLSSPVTKSFNRGEC.

### (2) Mutation of antibodies

For easily understanding the present invention, the present invention is illustrated with the nanobody 7D12 and the monoclonal antibody Trastuzumab as examples. It should be noted that the mutated antibodies of the present invention are not limited to the following antibodies or mutation sites, but the present invention is also applicable to mutation of other sites of other antibodies, and the mutation does not affect the performance of antibody-drug conjugates of the present invention.

### ① Mutation of nanobody 7D12

In this example, a single-domain nanobody 7D12 of an anti-Epidermal Growth Factor Receptor (EGFR) was chosen as a model antibody. Wherein, the 7D12 antibody has the following characteristics: (1) 7D12 displays a high affinity to EGFR and can induce internalization upon binding to EGFR; (2) 7D12 cannot activate antibody-dependent cell-mediated cytotoxic effects and complement-dependent cytotoxic effects due to the lack of Fc fragment, thus simplifying the interpretation of protein modifications in the cytotoxicity of antibody-drug conjugates. To prepare site-specific labeled antibody conjugates, Serine 85 (Ser85, S85) or Alanine 40 (Ala40, A40), Glycine 42 (Gly42, G42), Glycine 15 (Gly15, G15), Alanine 75 (Ala75, A75), or Glycine 66 (Gly66, G66) on the 7D12 antibody was mutated to cysteine (Cys, C). The amino acid sequences of the above mutated antibodies are shown below.

S85 mutated to C:

QVKLEESGGGSVQTGGSLRLTCAASGRTSRSYGMGWFRQAPGKEREFVSG
ISWRGDSTGYADSVKGRFTISRDNAKNTVDLQMNCLKPEDTAIYYCAAAA
GSAWYGTLYEYDYWGQGTQVTVSS.

A40 mutated to C:

QVKLEESGGGSVQTGGSLRLTCAASGRTSRSYGMGWFRQCPGKEREFVSG
ISWRGDSTGYADSVKGRFTISRDNAKNTVDLQMNSLKPEDTAIYYCAAAA
GSAWYGTLYEYDYWGQGTQVTVSS.

G42 mutated to C:

QVKLEESGGGSVQTGGSLRLTCAASGRTSRSYGMGWFRQAPCKEREFVSG
ISWRGDSTGYADSVKGRFTISRDNAKNTVDLQMNSLKPEDTAIYYCAAAA
GSAWYGTLYEYDYWGQGTQVTVSS.

G15 mutated to C:

QVKLEESGGGSVQTCGSLRLTCAASGRTSRSYGMGWFRQAPGKEREFVSG
ISWRGDSTGYADSVKGRFTISRDNAKNTVDLQMNSLKPEDTAIYYCAAAA
GSAWYGTLYEYDYWGQGTQVTVSS.

A75 mutated to C:

QVKLEESGGGSVQTGGSLRLTCAASGRTSRSYGMGWFRQAPGKEREFVSG
ISWRGDSTGYADSVKGRFTISRDNCKNTVDLQMNSLKPEDTAIYYCAAAA
GSAWYGTLYEYDYWGQGTQVTVSS.

G66 mutated to C:

QVKLEESGGGSVQTGGSLRLTCAASGRTSRSYGMGWFRQAPGKEREFVSG
ISWRGDSTGYADSVKCRFTISRDNAKNTVDLQMNSLKPEDTAIYYCAAAA
GSAWYGTLYEYDYWGQGTQVTVSS.

The preferred mutation site for 7D12 of the present invention is position 85, which was selected for mutation because: (1) it is in a middle region that is far away from the antigen-binding domain closed to the N-terminal of the antibody; (2) it is solvent accessible and can be further used for conjugation; and (3) it is located in the loop region, thus minimizing the possibility to perturb the quarternary structure of protein.

### ② Mutation of monoclonal antibody Trastuzumab

In this example, the monoclonal antibody Trastuzumab was selected as the model antibody. To obtain a site-specific labeled antibody, Alanine 121 (Ala121, A121) of the heavy chain on the Trastuzumab was mutated to cysteine (Cys, C).

The amino acid sequence of the above mutated antibody is shown below.

The amino acid sequence of the heavy chain of the mutated Trastuzumab is:
H-GAMMA1 (A121 mutated to C):

EVQLVESGGGLVQPGGSLRLSCAASGFNIKDTYIHWVRQAPGKGLEW
VARIYPTNGYTRYADSVKGRFTISADTSKNTAYLQMNSLRAEDTAVYYCSR
WGGDGFYAMDYWGQGTLVTVSSCSTKGPSVFPLAPSSKSTSGGTAALGCL
VKDYFPEPVTVSWNSGALTSGVHTFPAVLQSSGLYSLSSVVTVPSSSLGTQT
YICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPG.

The structure of the mutated antibody is suitable for the conjugation of drugs. However, it should be noted that the mutation sites and the mutated amino acids of the present invention are not limited to the above-mentioned positions, but in other embodiments, other positions can be mutated or mutated to other amino acids according to the needs of use.

### (3) Conjugation of antibody with small-molecule drugs

The above mutated antibodies are conjugated to small-molecule drugs with anti-tumor toxicity, including tubulin inhibitors and DNA damaging agents. Tubulin inhibitors can be Maytansinoids (DM1 and DM4), MMAF (monomethylaurethatin F), MMAE (monomethylaurethatin E), paclitaxel, taxanes, tubulysin, and the like. DNA damaging agents can be doxorubicin, calicheamicin, duocarmycin, camptothecin, pyrrolobenzodiazepines, and the like.

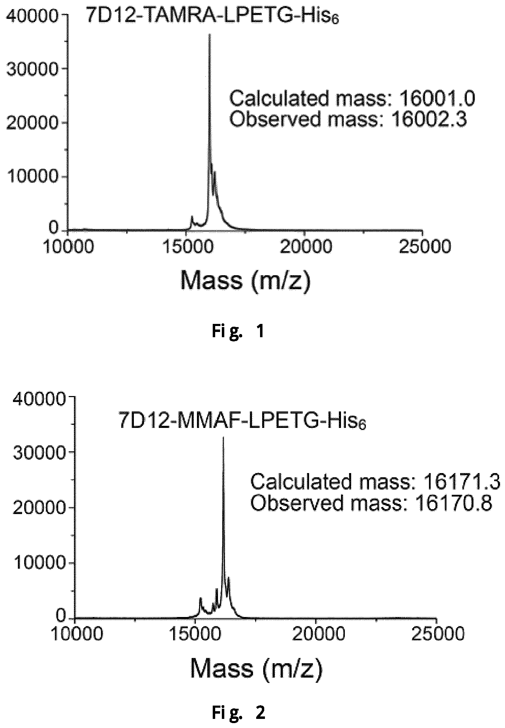

For the sake of illustration, the mutated 7D12 is chosen as the antibody and the maleimidecaproic acid-MMAF (Mc-MMAF) is selected as the small-molecule drug with anti-tumor toxicity in this example, however it should be understood that the protocols described below are also applicable to other antibodies and small-molecule drugs, and that MMAF is conjugated to the mutated 7D12 by orthogonal reaction of maleimide and sulfhydryl groups. The coupling of the small molecule drug is performed as follows:
First, the sulfhydryl group (-SH) on the mutated antibody 7D12 was reduced with 5 mM Tris(2-carboxyethyl)phosphine (TCEP; Thermo Fisher Scientific) for about 30 min at room temperature. The remained TCEP was removed by dialysis with a PBS buffer containing 1 mM EDTA, and then 5 eq of mc-MMAF was added to the protein solution. After incubation at 4°C overnight, the excess small-molecule drug was removed by buffer exchange in to PBS buffer to obtain the desired antibody conjugate. The molecular weight of the obtained protein mixture was determined by matrix-assisted laser desorption ionization time-of-flight mass spectrometry (MALDI-TOF MS). As shown in Fig. 2 of the MALDI-TOF mass spectrum of 7D12-MMAF-LPETG-His₆ described in this example, it can be seen that the mutated antibody 7D12 has completely coupled with MMAF to form 7D12-MMAF antibody-drug conjugates.

### (4) Labeling of antibody with fluorescent molecules

Preferably, to facilitate the monitor of the internalization of the antibody-drug conjugates, the antibody-drug conjugates of the present invention can also be labeled with a fluorescent molecule. The fluorescent molecule can be rhodamine, CY3 fluorescent dye (Cyanine 3), or Texas Red fluorescent dye.

For the convenience of illustrating the labeling method of the present invention, the 7D12 antibody is used as an example. Obviously, the labeling method of the present invention is not limited to the application on 7D12. The labeling method is also applicable to other antibodies.

In this example, the fluorescent molecule is maleimide-PEG3-tetramethylrhodamine (TAMRA), which is conjugated to the mutated 7D12 by the orthogonal reaction of maleimide and sulfhydryl groups. The fluorescent molecules were labeled as follows.

First, the -SH on the mutated 7D12 antibody was reduced with 5 mM TCEP (TCEP; Thermo Fisher Scientific) for about 30 min at room temperature. The remained TCEP was removed by dialysis with a PBS buffer containing 1 mM EDTA, and then 5 eq of maleimide-PEG3-TAMRA was added to the protein solution. After incubation at 4°C overnight, the excess small molecule was removed to obtain the desired antibody conjugate. The molecular weight of the obtained protein was determined by MALDI-TOF MS. As shown in Fig. 1 of the MALDI-TOF mass spectrum of 7D12-TAMRA-LPETG-His₆ described in this example, it can be seen that the mutated antibody 7D12 has been completely labeled with TAMRA.

### Example 2

### Modification with functional peptides

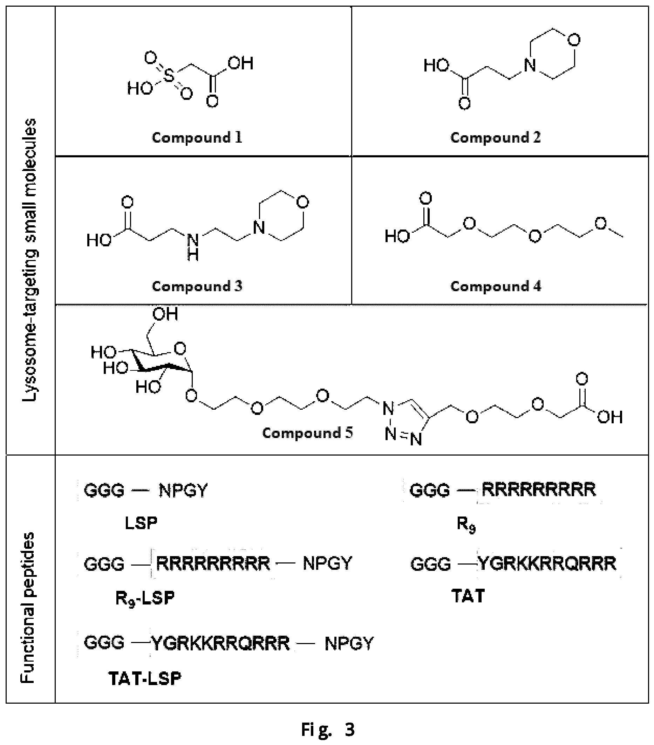

In one example, the antibody-drug conjugate of the present invention is modified with a functional peptide, which can be a lysosome-sorting peptide (LSP), a cell-penetrating peptide (CPP, e.g., TAT or R₉), or a combination of a lysosome-sorting peptide and a cell-penetrating peptide, TAT-LSP and R₉-LSP. The amino acid sequences of LSP, TAT, R₉, TAT-LSP, and R₉-LSP are shown in Fig. 3.

The lysosome-sorting peptide is one selected from NPXY, YXXØ, [DE]XXXL[LI], DXXLL, NPFXD, NPFXXD, wherein the X is any amino acid, the Ø is any amino acid with a bulky hydrophobic side chain, the N is asparagine, the P is proline, the Y is tyrosine, the E is glutamic acid, the L is leucine, the I is isoleucine, the F is phenylalanine, the D is aspartic acid, the [DE] is denoted as one of the D or the E, and the [LI] is denoted as one of the L or the I.

The cell-penetrating peptide is one selected from a cationic cell-penetrating peptide, an amphiphilic cell-penetrating peptide, and a hydrophobic cell-penetrating peptide.

The cationic cell-penetrating peptide and the amino acid sequence thereof is any one selected from:
Polyarginine, the amino acid sequence is:
   Rₙ, with n being an integer greater than or equal to 1;
   HIV-TAT, the amino acid sequence is:
      YGRKKRRQRRR;
      DPV1047, the amino acid sequence is:
         VKRGLKLRHVRPRVTRMDV;
         Penetratin, the amino acid sequence is:
            RQIKIWFQNRRMKWKK.

The amphiphilic cell-penetrating peptide and the amino acid sequence thereof are any of the following.

MPG, the amino acid sequence is:
GALFLGFLGAAGSTMGAWSQPKKKRKV ;

PVEC, the amino acid sequence is:
LLIILRRRIRKQAHAHSK;

MAP, the amino acid sequence is:
KLALKLALKALKAALKLA;

Pept-1, the amino acid sequence is:
KETWWETWWTEWSQPKKKRKV;

Transportan, the amino acid sequence is:
GWTLNSAGYLLGKINLKALAALAKKIL;

P28, the amino acid sequence is:
LSTAADMQGVVTDGMASGLDKDYLKPDD.

The hydrophobic cell-penetrating peptide and the amino acid sequence thereof are any of the following.

C105Y, the amino acid sequence is:
CSIPPEVKFNKPFVYLI;
PFVYLI, the amino acid sequence is:
   PFVYLI;
   Pep-1, the amino acid sequence is:
      SDLWEMMMVSLACQY.

The modification methods of functional peptide comprise the attachment to the C-terminal of antibody by using transpeptidase-mediated method and genetic recombination method. The method of functional peptide modification comprises the steps of:

### (1) Ligating a functional peptide to the C-terminal of antibody through transpeptidase

In order to diminish the impact of the attached functional peptide on the structure of the antibody, the functional peptide is ligated to the C-terminal of the mutated antibody using a transpeptidase. Two important elements are required to achieve the above attachment: (1) the addition of a transpeptidase recognition sequence (LPETG) to the C-terminal of the antibody by genetic engineering, and (2) the addition of an oligoglycine sequence (in this example, triglycine GGG) to the N-terminal of the functional peptide sequence.

### ① The mutated antibody is fused with a LPETG segment followed by a His₆ tag

Firstly, a segment of LPETG sequence and His₆ tag sequence were fused at the C-terminal of the mutated antibody 7D12-S85C, and the amino acid sequence after ligation was:

QVKLEESGGGSVQTGGSLRLTCAASGRTSRSYGMGWFRQAPGKEREFVSG
ISWRGDSTGYADSVKGRFTISRDNAKNTVDLQMNCLKPEDTAIYYCAAAA
GSAWYGTLYEYDYWGQGTQVTVSSLPETGLEHHHHHH.

Expression in *E.coli* BL21 (DE3) yielded 7D12-S85C-LPETG-His₆, which was purified with Ni-NTA beads. Briefly, the Ni-NTA beads were first equilibrated with equilibration buffer (buffer) (400 mM NaCl, 50 mM Tris-HCl pH 8.0, 20 mM imidazole), and was then loaded with the supernatant of cell lysate. The non-specific bound protein was removed with the equilibration buffer, and finally the desired protein was eluted with an elution buffer containing 200 mM imidazole. The protein concentration of the resultant solution was determined by Bicinchoninic Acid (BCA) protein Assay. The yield of the mutated protein is approximately the same as that of the wild-type protein.

### ② Functional peptide modification

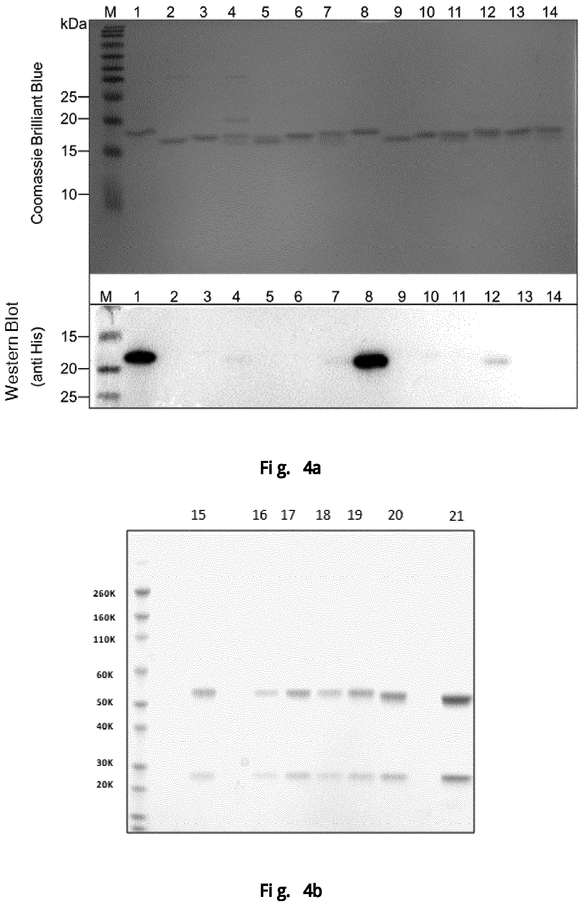

The transpeptidase of this example is the truncated fragment SrtA_{ΔN59} of transpeptidase A (Sortase A) from *Staphylococcus aureus.* The amino acid sequence of the SrtA_{ΔN59} is:
QAKPQIPKDKSKVAGYIEIPDADIKEPVYPGPATPEQLNRGVSFAEENESLD DQNISIAGHTFIDRPNYQFTNLKAAKKGSMVYFKVGNETRKYKMTSIRDV KPTDVGVLDEQKGKDKQLTLITCDDYNEKTGVWEKRKIFVATEVKLEHHH HH. The expression method was performed using the currently existing techniques. Meanwhile, the nucleophilic substrates (GGG-LSP, GGG-R₉, GGG-R₉-LSP, GGG-TAT, GGG-TAT-LSP) for the transpeptide reaction were synthesized using the standard solid-phase peptide synthesis method. Finally, the transpeptide reaction was carried out in SrtA_{ΔN59} working buffer (150 mM NaCl, 50 mM Tris-HCl pH 7.5) with 5 eq SrtA_{ΔN59}, 1 eq 7D12-TAMRA-LPETG-His₆ or 7D12-MMAF-LPETG-His₆, or Trastuzumab-TAMRA-LPETG-His₆ or Trastuzumab-MMAF-LPETG-His₆ and 10 eq of peptide substrate, and the reaction mixture was incubated overnight at room temperature. After the reaction, the reaction mixture was purified by Ni-NTA beads. Efficient cleavage of the His₆ tag by the enzymatic ligation afford no His₆-tagged antibody that could not be bound to the Ni column, while the unreacted antibody and SrtA_{ΔN59} were adsorbed on the Ni column. Finally, the reaction product was obtained by elution with low concentration of imidazole. The final recovered protein product was analyzed by sodium dodecyl sulfate-polyacrylamide gel electrophoresis (SDS-PAGE) and anti-His western blot, which showed no His₆-tagged antibody. In order to avoid any impact from the His₆ tag on antibody-drug conjugates, GGG was ligated to 7D12-TAMRA-LPETG-His₆, 7D12-MMAF-LPETG-His₆, Trastuzumab-TAMRA-LPETG-His₆ and Trastuzumab-MMAF-LPETG-His₆ using the above method to obtain 7D12-TAMRA-GGG, 7D12-MMAF-GGG, Trastuzumab-TAMRA-GGG and Trastuzumab-MMAF-GGG as controls. Fig. 4a shows the results of SDS-PAGE and His-tagged antibody immunoblotting assay (anti-His western blot) characterization of the 7D12-peptide conjugates, where the peptide conjugates corresponding to each lane are as follows: M: marker; 1: 7D12-TAMRA-LPETG- His₆; 2: 7D12-TAMRA-GGG; 3: 7D12-TAMRA-LSP; 4: 7D12-TAMRA-TAT; 5: 7D12-TAMRA-TAT-LSP; 6: 7D12-TAMRA-R₉; 7: 7D12-TAMRA-R₉-LSP; 8: 7D12-MMAF-LPETG -His₆; 9: 7D12-MMAF-GGG; 10: 7D12-MMAF-LSP; 11: 7D12-MMAF-TAT; 12: 7D12-MMAF-TAT-LSP; 13: 7D12-MMAF-R₉; 14: 7D12-MMAF-R₉-LSP. Fig. 4b shows the SDS-PAGE of Trastuzumab peptide conjugates, 15: Trastuzumab; 16: Trastuzumab-TAMRA-LSP; 17: Trastuzumab-MMAF-LSP; 18: Trastuzumab-TAMRA-R₉; 19: Trastuzumab-MMAF-R₉; 20: Trastuzumab-TAMRA-R₉-LSP; 21: Trastuzumab-MMAF-R₉-LSP.

### (2) Attachment of functional peptide to the C-terminal of antibody using genetic recombination method

### ① Plasmid construction

Plasmid pET28a-7D12-S85C-LPETG-His₆ was used as a template, the GAAACCTGTACTTCCAGGGA sequence for TEV protease cleavage was added at the N-terminal. Meanwhile, the fusion peptide sequence was added at the C-terminal to replace the LPETG-His₆ segment. The C-terminal was used for PCR reaction with a sense primer (5'-GGAATTCCATATGGAAAACCTGTACTTCCAGGGACAGGTGAAACTGG AGGAAAGCG-3') and an antisense primer (5'-CCGCTCGAGTCAATAGCCCGGGTTGCCGCTGCCTGCTAACGGTCACTTG GGTACC-3') to construct pET28a-7D12-S85C-Peptide.

### ② Protein expression

10 µl of *E. coli* BL-21 glycerol stock containing pET28a-7D12-S85C-Peptide plasmid was added to 10 ml of Luria broth (LB) medium (containing 50 µg/ml kanamycin) and incubated overnight in a constant temperature shaker (37°C, 220 rpm) to obtain seed solution for bulk expression. 10 ml of seed solution was transferred to 1 L of fresh LB medium (containing 50 µg/ml kanamycin) and incubated in a shaker at 37°C for around 8 h. The optical density value of the bacterial solution at 600 nm was read with a UV-Vis spectrophotometer, and when the OD600 reaches 0.5-0.6, the IPTG inducer (final concentration of 1 mM) was added followed by incubation at 25°C for another 16 h.

Purification and collection of 7D12-S85C-Peptide: the culture broth was placed in a floor-type high-speed centrifuge and the bacteria was collected by centrifugation using a horizontal rotor (4°C, 5000 rpm, 30 min). The supernatant was discarded, and the cell pellets were resuspended by adding appropriate amount of Ni-NTA Buffer A (approximately 15 ml of Ni-NTA Buffer A per liter of culture). The bacterial suspension is placed in an ice bath and the bacteria are lysed using an ultrasonic cell crusher (probe diameter 6 mm, power 260 W) until the suspension is no longer viscous and appears as a translucent homogeneous solution. The crushed solution was centrifuged at high speed using a fixed-angle rotor to remove uncrushed bacteria, bacterial residues, and insoluble proteins (4°C, 18,000 rpm, 30 min). The 7D12-S85C protein was purified using a nickel column, and the resin was rinsed with Ni-NTA Buffer A (approximately 20 times of the column volume). All the Ni-NTA Buffer A flowing through the nickel column was collected for the detection of the impurity that was not specifically bound to the nickel column. Subsequently, the desired proteins on the nickel column resin were eluted with Ni-NTA Buffer B containing various imidazole concentrations (20 mM, 50 mM, 100 mM, 200 mM, 400 mM, 800 mM) in a gradient elution, and each fraction was collected separately and analyzed by SDS-PAGE electrophoresis.

Based on the results of SDS-PAGE electrophoresis, the fractions containing relatively pure product were combined and transferred to protein concentration tubes, and the high-salt Ni-NTA Buffer containing imidazole was replaced with PBS (pH 7.4) containing 5 mM β-ME to ensure that the imidazole content in the protein solution was reduced to less than 1 mM, and the protein solution was changed four times and concentrated to 1.5 ml and the concentration of 7D12-S85C solution was measured using NanoDrop.

### (3) Protein conjugation with fluorescent or cytotoxic small molecules

Conjugation of 7D12-S85C-Peptide with Maleimide-PEG3-TAMRA: PBS (pH 7.4) containing 5 mM TCEP (a very potent thiol reducing agent that reduces disulfide bonds within or between proteins) was added to 7D12-S85C protein followed by incubation for 30 min at room temperature. The protein solution was then transferred to a protein concentration tube (cut-off value of 10 kDa) and exchanged (3800 rpm, 4°C) with PBS (pH 7.4) containing 1 mM EDTA. The protein after the buffer exchange was stored in 1.5-ml Eppendorf (EP) tubes and the concentration was determined by NanoDrop. Next, conjugation reaction was performed with a total reaction volume of 1 ml, and the final concentration of protein was 1 mg/ml, with maleimide-PEG3-TAMRA (dissolved with appropriate amount of DMSO) about five times the amount of protein. The fluorescent molecules were mixed with protein and incubated in a shaker at 4°C for 2 h (protected from light). After the reaction was completed, the fluorescent small-molecules were removed by PBS (pH 7.4) buffer exchange, and the protein concentration was measured by NanoDrop. The 7D12-TAMRA-labelled protein after reaction was stored in a refrigerator at -80°C.

Conjugation of 7D12-S85C-Peptide with mc-MMAF: PBS (pH 7.4) containing 5 mM TCEP was added to the 7D12-S85C protein followed by incubation for 30 min at room temperature. The protein solution was then transferred to a protein concentration tube (cut-off value of 10 kDa) and exchanged with PBS (pH 7.4) buffer containing 1 mM EDTA (3800 rpm, 4°C). The protein after the buffer exchange was stored in 1.5-ml EP tubes and the concentration was determined by NanoDrop. Next, conjugation reaction was performed with a total reaction volume of 1 ml, and the final concentration of protein was 1 mg/ml, with mc-MMAF (dissolved with appropriate amount of DMSO) five times the amount of protein. The cytotoxic molecules were mixed with protein and incubated in a shaker at 4°C for 2 h. After the reaction was completed, the small-molecule drug was removed through PBS (pH 7.4) exchange and the protein concentration was determined by NanoDrop. The 7D12-MMAF-conjugated protein after reaction was stored in a refrigerator at -80°C.

For easily understanding the present invention, 7D12-MMAF and Trastuzumab-MMAF in Example 1 have been chosen as examples in this embodiment for illustration, however the present invention is not limited thereto, as the modification method of this embodiment is also applicable to other antibody-drug conjugates formed by other cytotoxic small molecules with other antibodies.

It should be noted that when the functional peptide is conjugated to the C-terminal of the antibody using transpeptidase-mediated method, the present invention does not particularly limit the order of functional peptide modification and conjugate drugs, i.e., the mutated antibody can be modified by the functional peptide before coupling with small-molecule drug or coupled with small-molecule drug before modification by the functional peptide; the order of functional peptide modification and drug conjugation does not affect the performance of the antibody-drug conjugates of the present invention. When the functional peptide is conjugated to the C-terminal of the antibody using genetic recombination method, it is required in the present invention that the mutated antibody is to be modified with the functional peptide before coupling with the anti-tumor small-molecule drug.

### Example 3

### Modification of antibody-drug conjugates with a lysosome-targeting small molecule

In another example, the antibody-drug conjugates of the present invention can be modified with a lysosome-targeting small molecule in addition to the modification by attachment of a functional peptide to the C-terminal of the antibody.

The lysosome-targeting small molecule is a compound containing a pH-sensitive group or a compound containing a sugar group, wherein said pH-sensitive group is one selected from sulfonic acid group, a 4-morpholinyl group, 2-(2-morpholinoethylamino)ethyl group and polyethylene glycol group; the sugar group is one selected from glucose, mannose, mannose-6-phosphate, and galactose. The structure of the lysosome-targeting small molecule is shown below.
X-Y-Z;

Wherein,
X is selected from 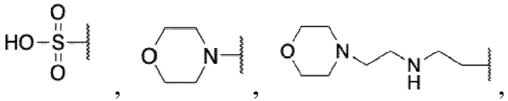 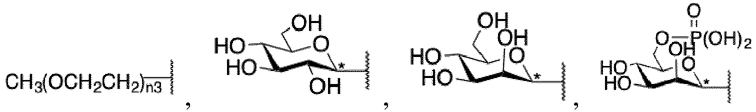 , and 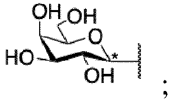
Y is selected from 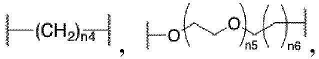 and 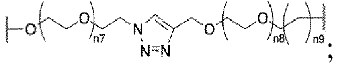
Z is selected from 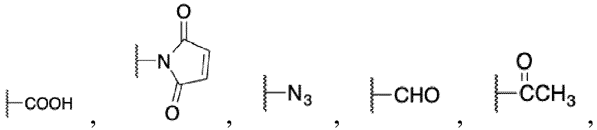 and 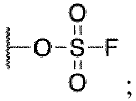
the wavy lines indicate the position of the linkage;
the asterisk indicates that the carbon atom at that position is a mixture of one or two of the differential isomers;
n3, n4 are integers greater than or equal to 1;
n5, n6, n7, n8, n9 are integers greater than or equal to 0;
n5 and n6 are identical or different;
n7, n8 and n9 are identical or different.

The lysosome-targeting small molecule is conjugated to the side chain of the amino acid of the antibody. The side chain of the amino acid is the side chain of a natural amino acid or a bioorthoganol group. The side chain of the natural amino acid is a lysine side chain or a cysteine side chain; the bioorthogonal group is an azide group, an alkynyl group, an aldehyde group, a ketone group, or a fluorosulfate groups that can undergo bio-orthogonal reaction. The bioorthoganol group can be introduced by genetic code expansion technique or enzymatic reaction.

For easily understanding the present invention, the present invention is illustrated taking the lysosome-targeting small molecules (1-9) in Fig. 3 as an example.

The specific steps for modification the antibody-drug conjugates by the lysosome-targeting small molecule of this embodiment are as follows.

In this example, the lysosome-targeting small molecules are conjugated to antibodies labeled by MMAF and TAMRA, respectively (7D12-MMAF and 7D12-TAMRA).

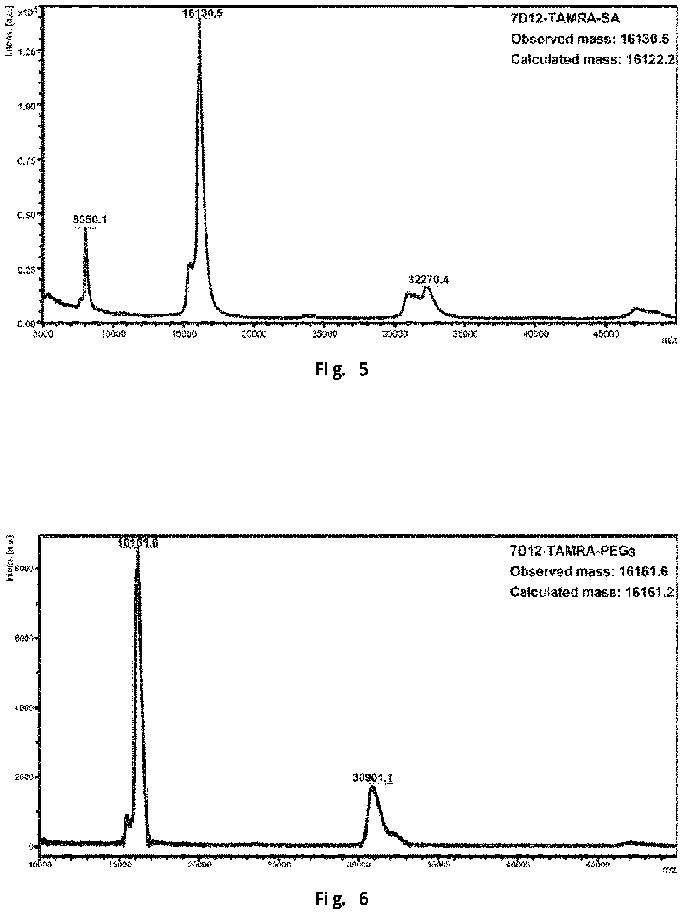

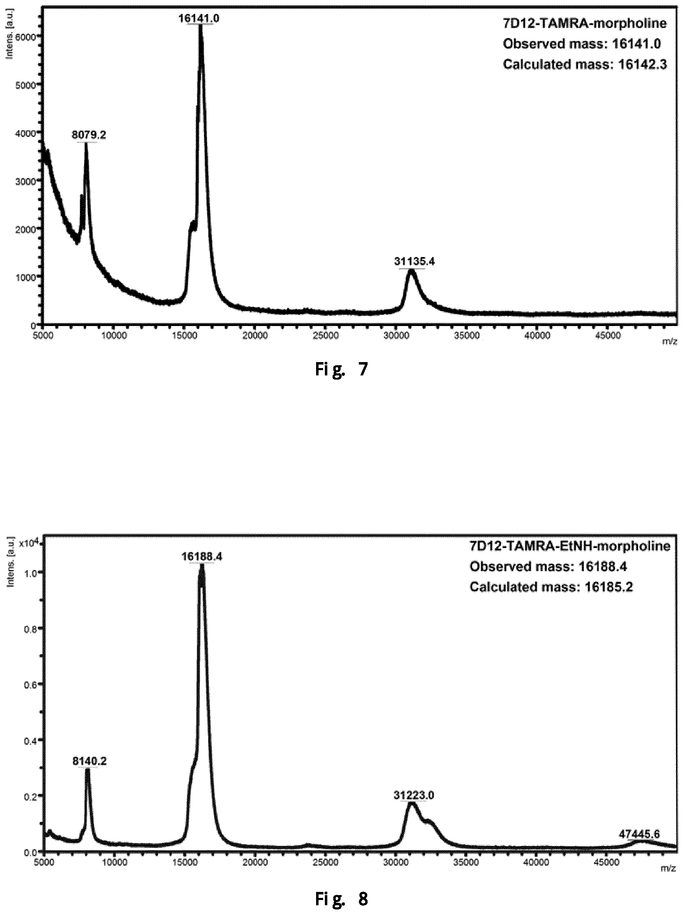

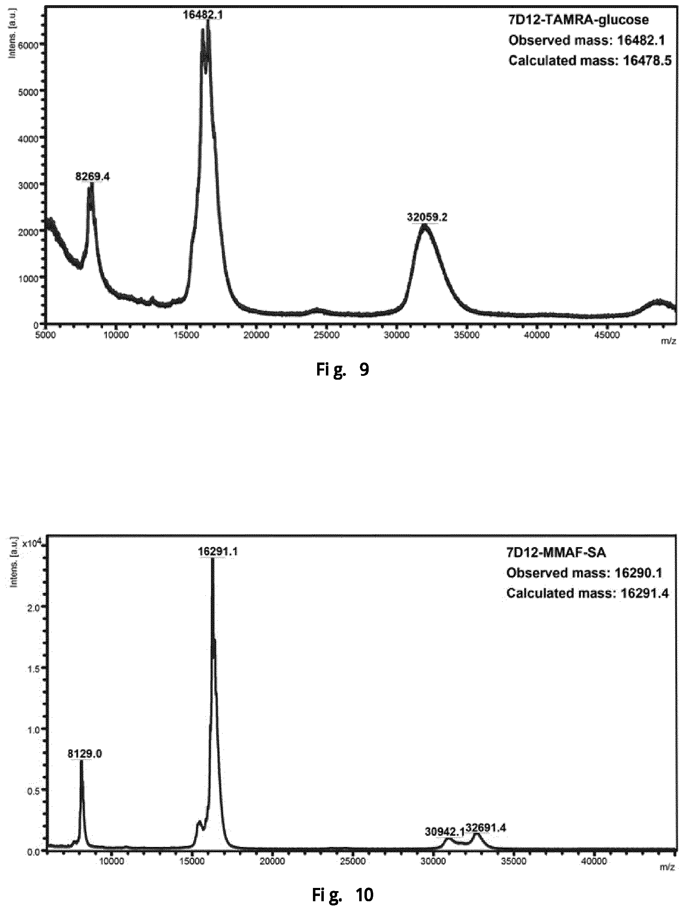

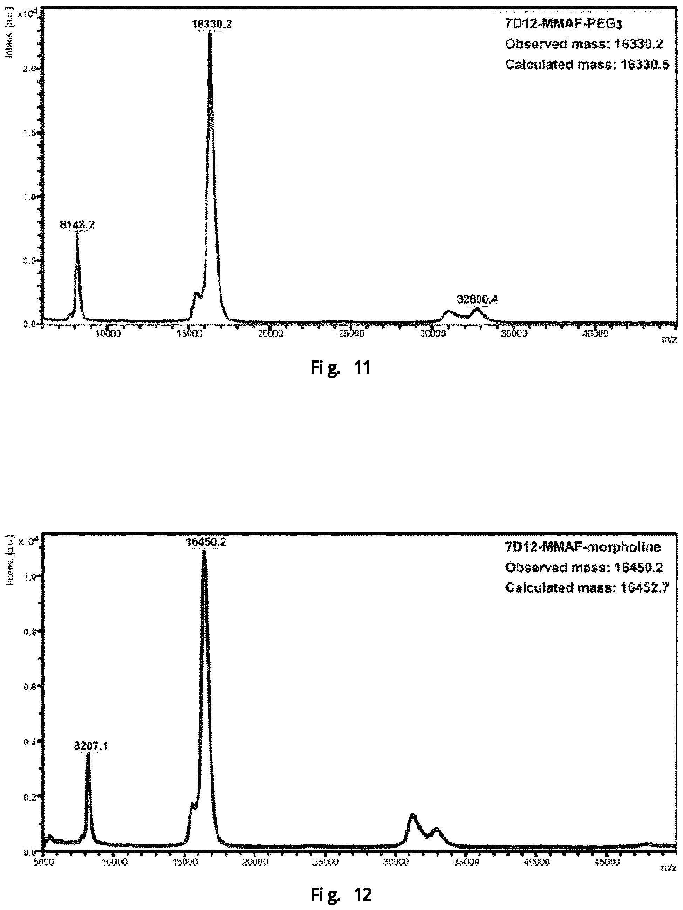

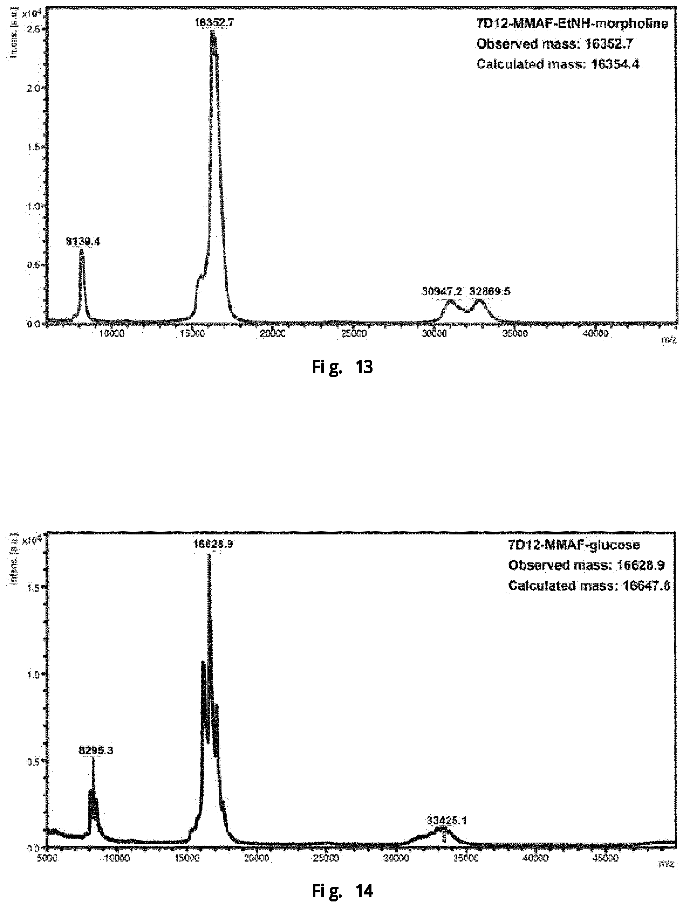

To attach the lysosome-targeting small molecules to the antibodies, the following steps were used: first, the small molecules were dissolved with DMF and activated by adding 2 eq of EDC/NHS, and the DMF was removed in a vacuum concentrator after 6 h of activation at RT. When the solvent was evaporated, PBS was added, and 7D12-TAMRA or7D12-MMAF was added for conjugation overnight at 4°C. The small molecules were then removed by ultrafiltration concentration, and the obtained protein was characterized by MALDI-TOF MS. The MALDI-MS results are shown in Figs. 5-14. According to the results in Figs. 5-14, a mixture of protein was obtained without site-specificity; however, each protein was conjugated to 1 small molecule on average. A total of 10 conjugates were finally obtained, including 7D12-MMAF-SA (Fig. 10), 7D12-MMAF-morpholine (Fig. 12), 7D12-MMAF-EtNH-morpholine (Fig. 13), 7D12-MMAF-PEG3 (Fig. 11), 7D12-MMAF-glucose (Fig. 14),7D12-TAMRA-SA (Fig. 5), 7D12-TAMRA-morpholine (Fig. 7), 7D12-TAMRA-EtNH-morpholine (Fig. 8), 7D12-TAMRA-PEG3 (Fig. 6) and 7D12-TAMRA-glucose (Fig. 9).

It should be noted that the present invention does not specifically limit the order of lysosome-targeting small-molecule modification and drug coupling, i.e., the mutated antibodies of the present invention can be modified by lysosome-targeting small molecules before coupling with small-molecule drugs, or coupled with small-molecule drugs before modification by lysosome-targeting small molecules; the order of lysosome-targeting small-molecule modification and drug conjugation does not affect the performance of the antibody-drug conjugates of the present invention.

### Example 4

In this example, flow cytometry is used to monitor the internalization of the antibody-drug conjugates of the present invention modified by functional peptides and modified by lysosome-targeting small molecules, respectively. The procedure comprises the following steps:

### (1) Cell culture

A431, BT474, MCF-7, HEK293T cell lines (available from Shanghai Cell Bank, Chinese Academy of Sciences (source: ATCC)) were used, and the cells were cultured in DMEM (Gibco, USA) medium supplemented with 10% fetal bovine serum plus penicillin (100 U/mL)-streptomycin (100 g/mL). All cells were cultured at 37°C in a humidified environment containing 5% CO₂. The cells were morphologically normal and in good growth condition.

### (2) The Internalization of the modified antibody-drug conjugates by flow cytometry:

In addition to the specificity, selectivity and pharmacokinetic properties of antibody itself, the efficacy of ADCs is also dependent on the intracellular trafficking kinetics of the antigen. By flow cytometry, the internalization of 7D12conjugates modified by the fluorescent molecule TAMRA was monitored at various time points.

The modified 7D12 conjugates (7D12-TAMRA, 7D12-TAMRA-LSP, 7D12-TAMRA-R₉, 7D12-TAMRA-R₉-LSP, 7D12-TAMRA-TAT, 7D12-TAMRA-glucose) were co-incubated with A431 cells, and then diluted with serum-free medium to a final concentration of 2 µM. Subsequently, the bound antibodies on the cell membranes were removed through acid wash and quantified by flow cytometry at different time points.

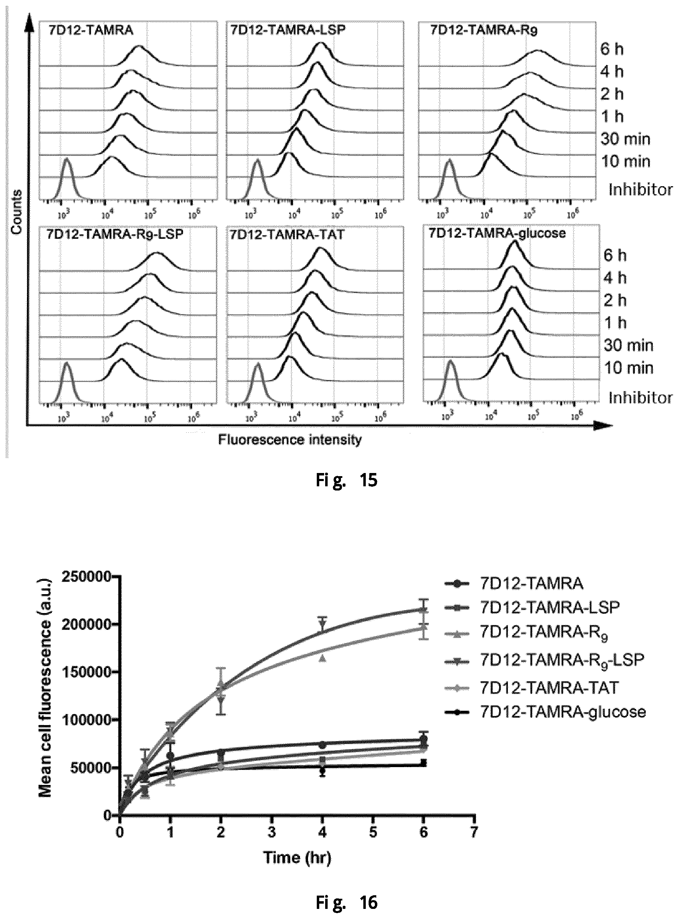

The analysis by flow cytometry and the analysis of the mean cell fluorescence values clearly showed that the different 7D12 conjugates had similar internalization endpoints and reached the endpoint at 4 hours. Therefore, we chose to observe their co-localization with lysosomes after 4 h of incubation in the subsequent experiments. In particular, the R₉-modified conjugates had a significantly stronger and faster internalization ability than those without the modification. In contrast, several other functional peptides show relatively small peak-shift at the same time, which also indicates that fewer antibody-drug conjugates are internalized into the cells with lower mean cell fluorescence values, after which a plateau is reached. In addition, there was no difference between R₉ and R₉-LSP, demonstrating that the attachment to R₉ had a direct and positive effect on cellular uptake and internalization. The test results are shown in Fig. 15 and Fig. 16. Fig. 15 shows the characterization of the internalization properties of the 7D12 conjugates, where representative flow cytometry plots show the internalization of the 7D12 conjugates at different time points. Fig. 16 shows the mean fluorescence intensity for the 7D12 conjugates internalized into A431 cells over time. Data are expressed as mean ± SEM (n = 3).

### Example 5

### Study of lysosome-targeting efficiency of antibody-drug conjugates modified by functional peptides and antibody-drug conjugates modified by lysosome-targeting small molecules:

An ADC is usually trafficking and processed in an endocytosis pathway specified as follows: an ADC binds to the tumor antigen on the cell surface, followed by internalization into endosomes, which subsequently mature and fuse with lysosomes. In lysosomes, the release of the cytotoxic drug can be achieved either through the degradation of the cleavable linker or catabolism of the entire antibody by proteases (e.g., cathepsin B). The released drug subsequently passes across the lysosomal membrane and reaches the cytoplasm to bind to its target, such as tubulin or DNA, ultimately inducing cell death. Therefore, whether an ADC can enter the lysosome after internalization or whether it can accumulate in the lysosome is critical to its efficacy.

Based on the internalization kinetics observed by the above flow cytometry assay, the time-point of 4 h was chosen as the endpoint to observe the degree of co-localization of the modified antibody with lysosomes. Followed by co-incubation for 4 hours, modified 7D12-conjugates and Trastuzumab-conjugates were observed by confocal laser scanning microscopy to visualize lysosomal trafficking of antibody conjugates. Lysosomes in cells were stained with a lysosome marker of green fluorescence (LysoTracker Green), and 7D12-conjugates and Trastuzumab-conjugates were labeled with TAMRA of red fluorescence. All images were scaled and taken in an identical condition. In the quantitative analysis, the same threshold value was set for all images.

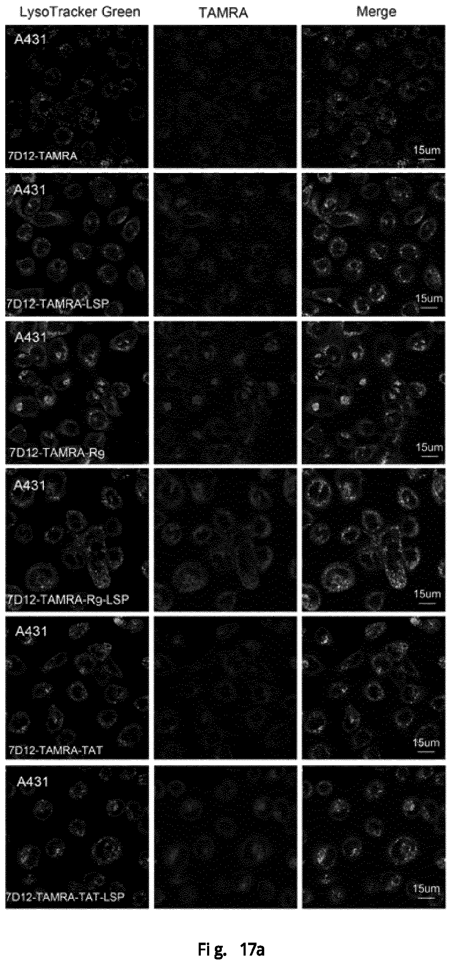

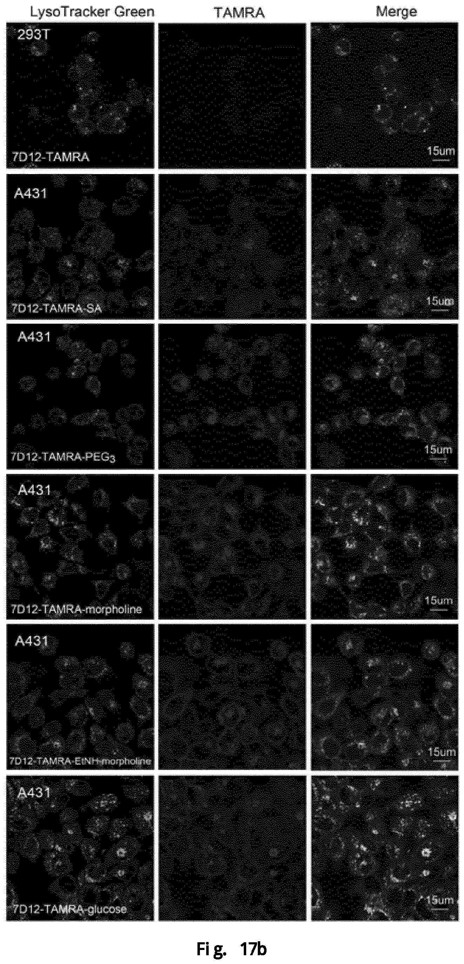

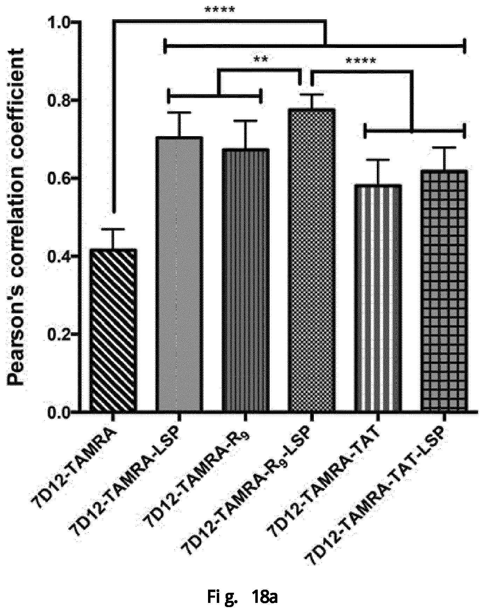

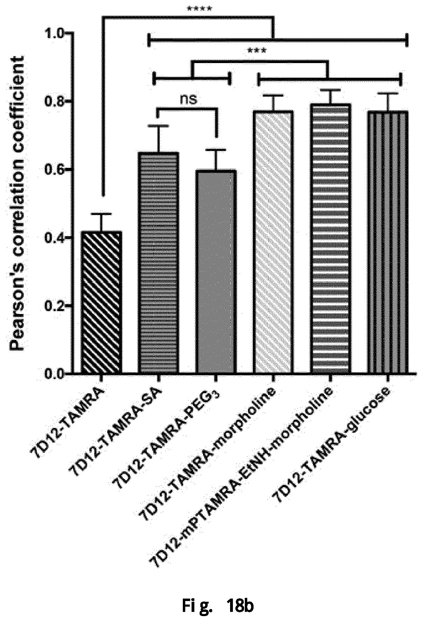

The confocal imaging results of the 7D12-conjugates are shown in Figs. 17a and 17b, which demonstrate the internalization and lysosomal trafficking of 7D12-conjugates. Specially, Fig. 17a shows the confocal microscopy images of 7D12-TAMRA, 7D12-TAMRA-LSP, 7D12-TAMRA-R₉, 7D12-TAMRA-R₉-LSP, 7D12-TAMRA-TAT, and 7D12-TAMRA-TAT-LSP, where the left and middle column show the signals from lysosomes and the 7D12-conjugates, respectively, and the right column shows the merged images of the left and middle columns. Fig. 17b shows the confocal microscopy images of 7D12-TAMRA, 7D12-TAMRA-SA, 7D12-TAMRA-PEG3, 7D12-TAMRA-morpholine, 7D12-TAMRA-EtNH-morpholine, 7D12-TAMRA-glucose, wherein the left and the middle columns show the signals from lysosomes and 7D12-conjugates, and the right column shows the merged images of the left and the middle columns. Fig. 18a shows the Pearson's correlation coefficients between the conjugates and lysosomes quantified and analyzed from Fig. 17a, and Fig. 18b shows the Pearson's correlation coefficients between the conjugates and lysosomes quantified and analyzed from Fig. 17b, as mean ± SEM. Error bars represent SEM (n = 20). Ns: not significant. ^{∗∗}p < 0.01; ^{∗∗∗}p < 0.001; ^{∗∗∗∗}p < 0.0001.

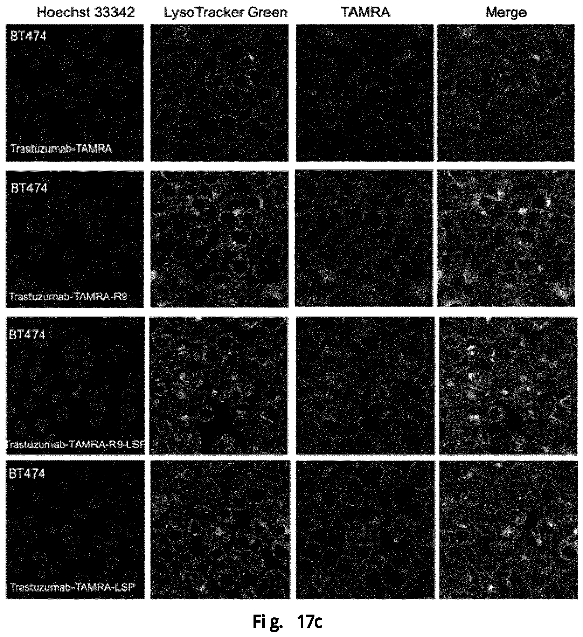

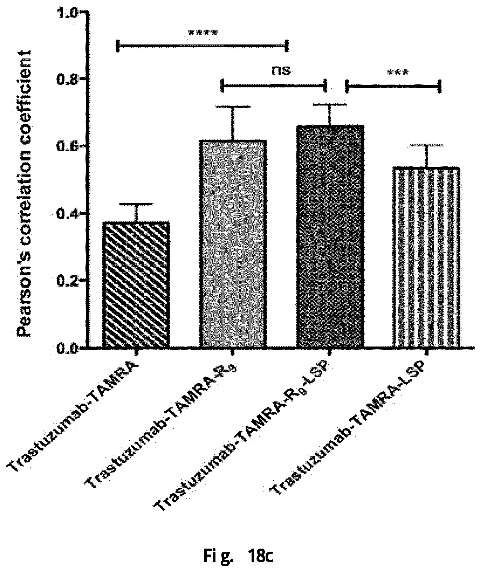

Trastuzumab-conjugates were also stained with Hoechst 33342 for the lysosomal trafficking study. The confocal imaging results of Trastuzumab-conjugates are shown in Fig. 17c, which shows the internalization and lysosomal trafficking of Trastuzumab-conjugates (Trastuzumab-TAMRA, Trastuzumab-TAMRA-R₉, Trastuzumab-TAMRA-R₉-LSP and Trastuzumab-TAMRA-LSP). The first, second and third column shows the signals from cell nuclei, lysosomes and Trastuzumab-conjugates, and the fourth column is the merged images of the first, second and third columns. Fig. 18c shows the Pearson's correlation coefficients between the conjugates and lysosomes quantified and analyzed from Fig. 17c, as mean ± SEM. Error bars represent SEM (n = 20). Ns: not significant. ^{∗∗∗}p < 0.001; ^{∗∗∗∗}p < 0.0001.

7D12-conjulates show antigenic specificity for EGFR. In the negative control HEK293T cell line with low expression of EGFR, 7D12 conjugates did not show specific binding at the cell membrane. Second, the attachment of R₉-LSP caused a substantial increase in co-localization of 7D12-conjugates with lysosomes in living cells. There was a significant difference in the degree of co-localization between the CPP-modification and the modification with the combination of CPP and LSP. It is shown that the CPP facilitates the internalization of antibody into the cell while the LSP helps to increase the accumulation of antibody in lysosomes. In addition, a few small-molecule-modified conjugates have also been shown to be well lysosome-targeting, including EtNH-morpholine-modified and glucose-modified conjugates. It has not been reported to apply the above small molecules to protein modification for lysosomal targeting.

### Example 6

### Quantitative analysis of the effect of modification by functional peptides and by lysosome-targeting small molecules was performed to quantify the internalization of antibody-drug conjugates:

After the co-localization study of antibody-conjugates with lysosomes in Example 4, the 7D12-conjugates internalized into cells was quantified by values. Such a quantification can reflect the variation of antibody internalization with different modifications, and can be used to explain the lysosomal trafficking behavior of antibody-drug conjugates after internalization.

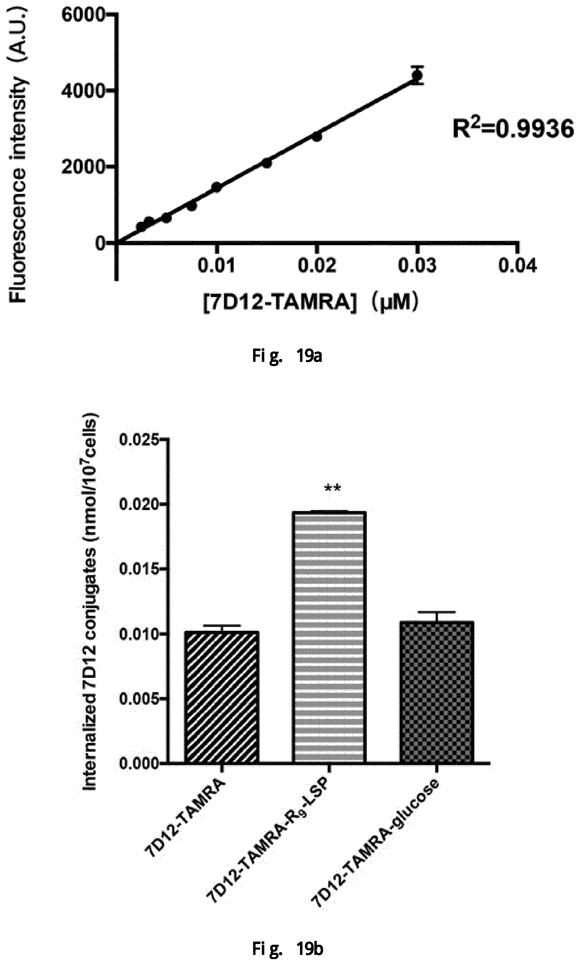

To quantify the antibody-drug conjugates internalized into the cells, a fluorescence assay was conducted, where the linear calibration curve for 7D12-TAMRA was shown in Fig. 19a. The test results showed that the standard curve was highly linear (R² = 0.9936) for conversion of raw fluorescence intensity to 7D12-TAMRA concentration. Quantification of modified-7D12 internalization was performed by incubating 7D12-conjugates with A431 cells in 12-well plates at a final concentration of 2 µM for 4 h. After acid washing of cell surface bound antibodies, cells were lysed to obtain the supernatant followed by the performance of fluorescence measurements. Using the standard calibration curve, the amount of antibody internalized into the cells was calculated and the results are shown in Fig. 19b, with data expressed as mean ± SEM (n = 3), ^{∗∗}P < 0.01.

The results showed that 7D12 modified with R₉-LSP had a higher internalization concentration compared to unmodified-7D12, which again demonstrated that R₉ increased the internalization of antibodies. On the other hand, the modification by glucose did not show much impact on the internalization of 7D12.

### Example 7

### Study on internalization mechanism of antibody-drug conjugates modified by functional peptides and antibody-drug conjugates modified by lysosome-targeting small molecules:

Endocytosis of cells is a complex mechanism involving different pathways and large networks of protein-protein and protein-lipid interactions. The most extensively studied and best characterized pathway is clathrin-mediated endocytosis, which is initiated from the formation of clathrin-coated pits on the plasma membranes followed by the development of clathrin-coated vesicles. Caveolin-mediated endocytosis, is a non-classical endocytic pathway independent of clathrin. Previous studies suggest that clathrin-mediated endocytosis can facilitate the fusion with lysosomes, thus ultimately leading to digestion of endocytosed materials in the acidic and proteolytic environment of lysosomes. In contrast, caveolin-mediated endocytosis tends to redirect the early membrane-bound vesicles to fuse with the Golgi apparatus or endoplasmic reticulum, where parts of the endocytosed materials can be transported out of cells.

In this example, firstly, to test whether the cellular uptake of 7D12 conjugates is energy-dependent, cells were incubated with 7D12-conjugates at 4 °C compared with 37 °C. The uptake of both functional-peptide-modified 7D12-conjugates and small-molecule-modified 7D12-conjugates were significantly inhibited at 4 °C, indicating that the internalization of 7D12-conjugates was ATP-dependent. Then, to further characterize the endocytosis mechanism, cells were pretreated with endocytosis inhibitors. The cationic amphiphilic drug chlorpromazine (CPZ) was used to disrupt clathrin-mediated endocytosis by inhibiting the recruitment of clathrin. N,N-ethylisopropylamide (EIPA), a Na⁺ /H⁺ exchange inhibitor that induces cholesterol chelation at the plasma membrane, is used to block macropinocytosis. Nystatin is used to disrupt the caveolin-mediated endocytosis. Cells were co-incubated with different 7D12-conjugates after treatment with different inhibitors at the recommended concentrations of use for optimal incubation time. The internalization behaviors were compared with the untreated group to determine the predominant mode of internalization.

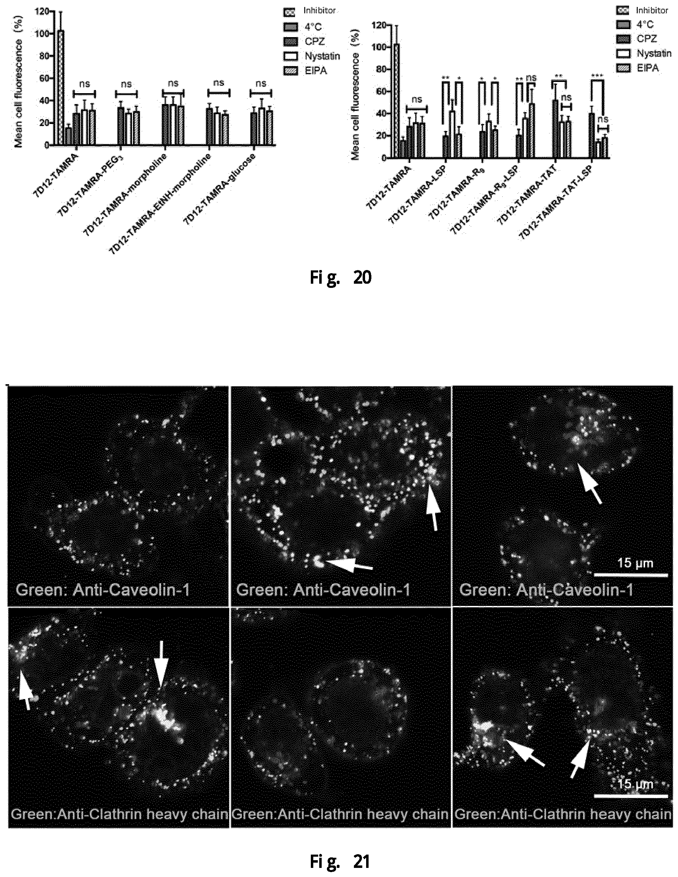

Each of the three inhibitors significantly inhibited the internalization of 7D12-conjugates. There was no significant difference between these inhibitors in the internalization of 7D12-TAMRA, 7D12-TAMRA-LSP and 7D12-TAMRA-morpholine. However, 7D12-TAMRA-R₉-LSP uptake was mainly inhibited by CPZ, while 7D12-TAMRA-TAT-LSP was mainly inhibited by Nystatin. The test results are shown in Fig. 20, which shows the effect of temperature and endocytosis inhibitors on the internalization of 7D12-conjugates. The uptake of 7D12-TAMRA-R₉-LSP and 7D12-TAMRA-TAT-LSP relies mainly on clathrin-mediated endocytosis and caveolin-mediated endocytosis, respectively, which is consistent with the results of lysosomal trafficking study.

### Example 8

### Studies on intracellular trafficking of antibody-drug conjugates modified by functional peptides and antibody-drug conjugates modified by lysosome-targeting small molecules:

Caveolin-mediated endocytosis is thought to be one of the possible mechanisms responsible for the resistance of antigen-expressing cells to ADCs. It is suggested in this study that internalization induced by TAT-modification is mainly via the caveolin-mediated endocytosis pathway. It is also indicated that LSP is internalized by binding of clathrin and adaptor protein AP2. Therefore, this example evaluates the co-localization of functional-peptide-modified 7D12-conjugates with either caveolin-1 or clathrin.

First, A431 cells were incubated with 7D12-conjugates using rabbit-derived antibodies against clathrin or caveolin-1 as primary antibodies. FITC-labeled anti-rabbit secondary antibodies were then incubated for immunofluorescence analysis.

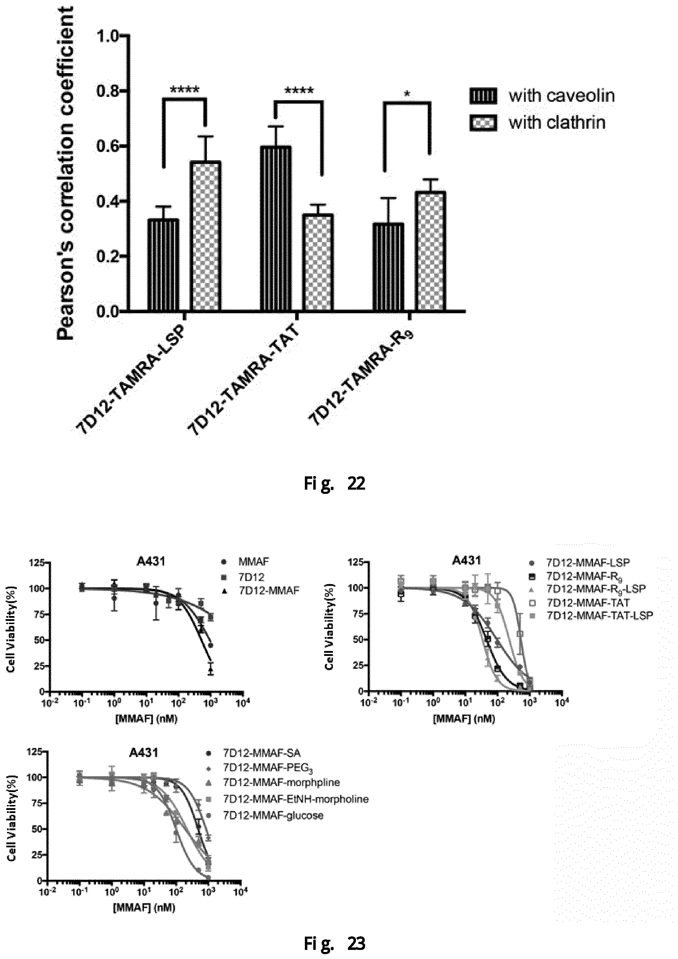

Fig. 21 shows co-localization of 7D12-conjugates (7D12-TAMRA-LSP, 7D12-TAMRA-TAT and 7D12-TAMRA-R₉) with clathrin or caveolin-1, where cell nuclei were stained with Hoechst 33342. The result showed that 7D12-TAMRA-LSP did not co-localize with caveolin-1, but did partially co-localize with clathrin. In contrast, 7D12-TAMRA-TAT was found to co-localize with caveolin-1, instead of clathrin. Differently, 7D12-TAMRA-R₉ co-localized with both clathrin and caveolin-1. Fig. 22 shows the Pearson's correlation coefficients between 7D12-conjugates (7D12-TAMRA-LSP, 7D12-TAMRA-TAT and 7D12-TAMRA-R₉) with clathrin or caveolin-1 (Pearson's correlation coefficients, mean ± SEM). Error bars represent SEM (n = 20). ^{∗}P < 0.05; ^{∗∗∗∗}P < 0.0001. This indicates that both clathrin-mediated endocytosis and caveolin-mediated endocytosis are involved in the internalization of 7D12-TAMRA-R₉.

### Example 9

### In vitro studies on the therapeutic effects of antibody-drug conjugates modified by functional peptides and antibody-drug conjugates modified by lysosome-targeting small molecules:

(1) The cytotoxicity of lysosome-targeting ADCs was studied by a 2D tumor cell model, and the steps are as follows:
   Human epidermal cancer cells A431 were selected to assess the ability of 7D12-MMAF conjugates to inhibit cancer cell proliferation. 2D tumor cell model was used, where ADCs of varying concentrations were added to a 96-well plate seeded with A431 cells and treated for 96 h. The cell viability was examined using Cell Counting Kit-8 (CCK-8).

The results of Fig. 23 (in vitro cytotoxicity of 7D12-conjugates assessed by 2D tumor cell model) shows that A431 cells were treated with MMAF, 7D12 or 7D12-MMAF conjugates for 96 hours. Cell viability was plotted versus the concentration of MMAF, 7D12 or the tested conjugates), where the modified 7D12-MMAF conjugates exhibited a significant killing activity. The results are consistent with lysosomal trafficking experiments. In general, 7D12 conjugates with stronger lysosome-targeting ability showed higher cytotoxicity against cancer cells. Notably, 7D12-MMAF-R₉-LSP showed the highest potency with an EC₅₀ value of 53.74 nM. 7D12-MMAF-EtNH-morpholine modified by small molecules had the lowest EC₅₀ of 117.9 nM. The unmodified 7D12 was used as an control antibody in the A431 cell line and no significant cytotoxicity was observed. In the negative control HEK293T cell line, 7D12-MMAF-R₉-LSP displayed certain cytotoxicity. This could be due to the cell-penetrating peptide (CPP), which possesses strong capacity to carry cargos into the cells, thus resulting in some non-specific killing. Confocal laser scanning microscopy study showed that the 7D12-conjugates modified with R₉ were internalized into negative cells after incubation in 6 hours or even longer. Therefore, the non-specific cytotoxicity caused by CPP modification should be considered and further optimized for application.

The EC₅₀ values (nM) of MMAF, 7D12 or the tested conjugates in A431 cells are shown in Table 1 and the data are expressed as mean ± SEM (n = 3).

**Table 1**

| | EC₅₀ ( nM ) | 7D12-MMAF-peptides | EC₅₀ ( nM ) | 7D12-MMAF-small-molecule | EC₅₀ ( nM ) |
|---|---|---|---|---|---|
| MMAF | 954.9±289 | 7D12-MMAF- | 95.49±11.5 | 7D12-MMAF- | 482.3±52.7 |
| | | LSP | | SA | |
| 7D12 | n.a. | 7D12-MMAF-R₉ | 45.72±5.88 | 7D12-MMAF-PEG3 | 852.0±125 |
| 7D12-MMAF | 612.5±88.9 | 7D12-MMAF-R₉-LSP | 34.13±3.04 | 7D12-MMAF-morpholine | 258.0±82.3 |
| | | 7D12-MMAF-TAT | 531.7±54.3 | 7D12-MMAF-EtNH-morpholine | 216.3±33.3 |
| | | 7D12-MMAF-TAT-LSP | 231.5±44.6 | 7D12-MMAF-glucose | 100.2±10.8 |

As shown in Table 1, the modified 7D12-MMAF conjugates exhibited higher efficiency in cell killing compared to MMAF and 7D12-MMAF. In general, the cytotoxicity of the modified 7D12-MMAF conjugates was correlated with their internalization and lysosomal targeting ability. For functional-peptide-modified 7D12-conjugates, 7D12-MMAF-R₉-LSP exhibited the highest potency, followed by 7D12-MMAF-R₉ and then 7D12-MMAF-LSP. Conjugates modified with TAT or TAT-LSP demonstrated a lower cell killing potency compared to conjugates modified with LSP, R₉, or R₉-LSP For lysosome-targeting-small-molecule-modified 7D12 conjugates, 7D12-MMAF-R₉-glucose provided the highest inhibitory activity against cancer cell growth, followed by 7D12-MMAF-EtNH-mophorline. There is no significant difference in the cell killing potency among 7D12 conjugates modified with sulfonic acid, PEG and morpholine.

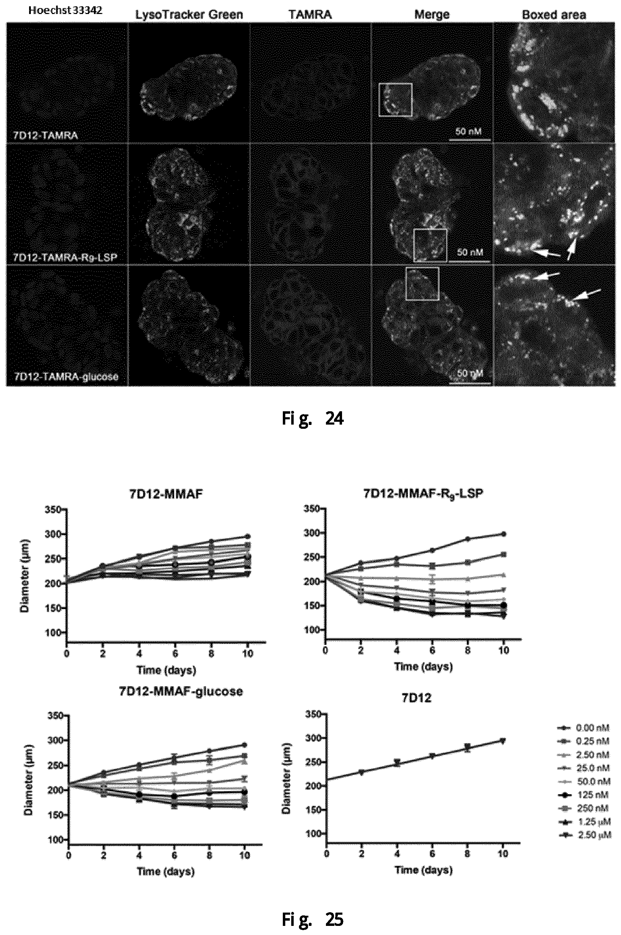

(2) The antitumor activity of lysosome-targeting ADCs was observed by a 3D tumor spheroid model, and the steps are as follows:
In addition to the monolayer tumor model for in vitro toxicity assessment, the permeability and efficacy of ADCs were further assessed using a 3D spheroid tumor model: the permeability and lysosome-targeting ability in 3D tumor spheroids was determined after 6 h of incubation with various 7D12-TAMRA conjugates. All tumor spheroids were approximately 100 µm in diameter. Fig. 24 shows the antitumor activity of 7D12-conjugates as assessed by a 3D tumor spheroid model. The images are A431 tumor spheroids treated with 7D12-TAMRA, 7D12-TAMRA-R₉-LSP, and 7D12-TAMRA-glucose, from top to bottom, respectively. The unmodified 7D12-TAMRA diffused only on the surface of the A431 tumor spheroid, instead of penetrating into the core of spheroids, and few conjugates were found to co-localize with lysosomes. 7D12-TAMRA-R₉-LSP showed higher permeability to A431 spheroids. 7D12-TAMRA-glucose shows only slightly less permeability than 7D12-TAMRA-R₉-LSP. Both of them displayed excellent lysosome-targeting ability.

Next, the viability assay was performed with the 3D tumor spheroid model. Three cell lines with EGFR expression level from high to low were selected: A431, MCF-7, and HEK293T. 3D spheroids with a symmetrical and spherical morphology of approximately 200 µm in diameter were added with modified ADCs. Specifically, the 3D spheroids were incubated in medium containing ADCs (0.25 nM to 2.5 µM) for 10 days while the size of the spheroids was measured daily.

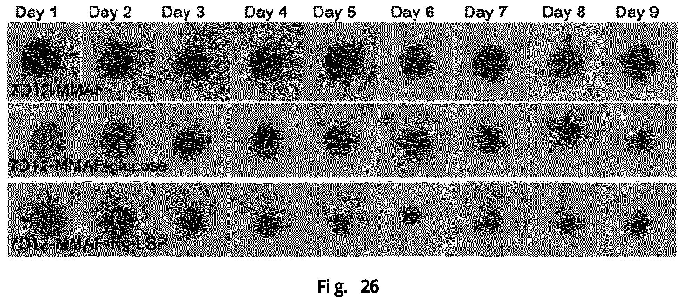

The changes in the mean diameter of the tumor spheroids treated with 7D12-conjugates can be seen in Figs. 25-26. Fig. 25 shows the inhibition of tumor spheroid growth under 7D12-conjugates of varying concentrations, where the data are expressed as mean ± SEM (n = 3). Fig. 26 shows the A431 spheroid morphology with the treatment of 2.5 µM 7D12-conjugates over nine days. 7D12-MMAF-R₉-LSP displayed the strongest growth inhibition on A431 tumor spheroids compared to 7D12-MMAF. Also, 7D12-MMAF-glucose showed a stronger growth inhibition of tumor spheroids. The above results are consistent with the results observed in the 2D tumor culture model. In addition to A431 cells with high expression of EGFR, MCF-7 and HEK293T were used to study the specificity of ADCs. The growth of MCF-7 spheroids with moderate EGFR expression was slightly inhibited while the growth of HEK293T spheroids was unaffected upon the treatment of ADCs. These results again demonstrate the ADCs exhibited significant anti-tumor activity and specificity in a 3D tumor spheroid model, which mimic the in vivo tumor development and behavior, and the lysosome-targeting modification can significantly the efficacy of ADCs.

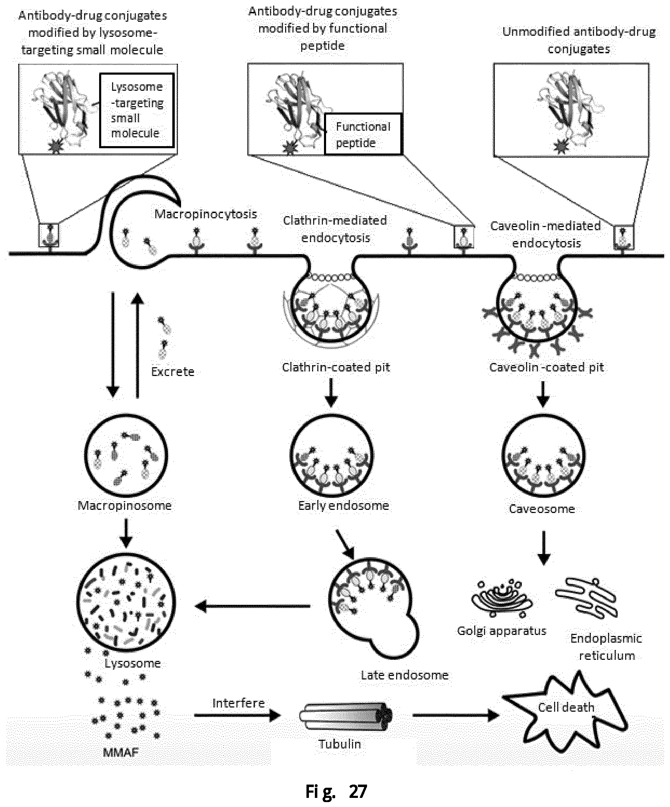

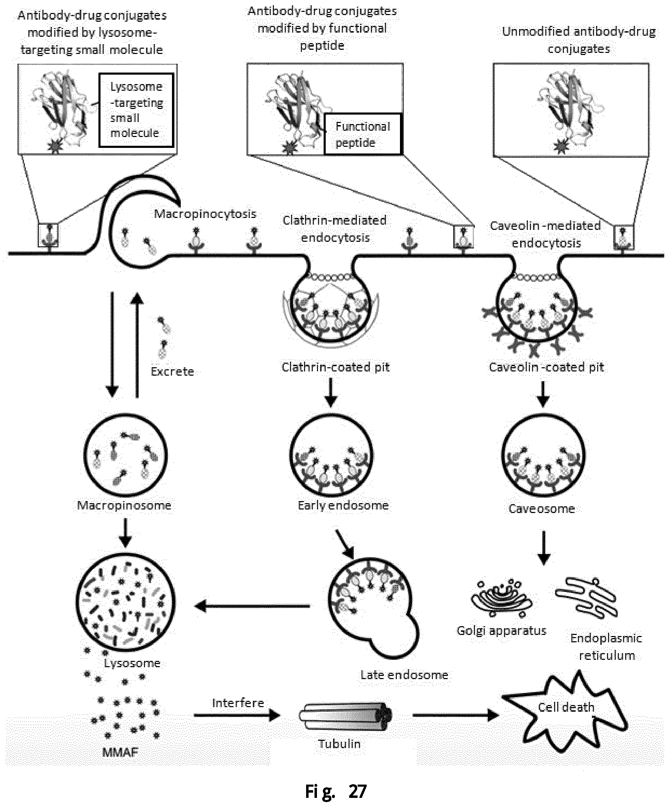

The above tests showed that lysosome-targeting antibody-drug conjugates (ADCs) were prepared by two types of modification methods: (1) attachment of a functional peptide to the C-terminal of the antibody; and (2) conjugation of a lysosome-targeting small molecule to the antibody. Both functional peptide modification and lysosome-targeting small molecule modification can enhance the lysosome-targeting activity of ADCs. The ADCs modified with the R₉-LSP functional peptide showed significantly enhanced internalization capacity, faster internalization rate and higher lysosomal trafficking activity. Mechanistic studies showed that ADCs modified with the R₉-LSP functional peptide increased clathrin-mediated endocytosis, while modification of lysosome-targeting small molecules altered the intracellular trafficking of ADCs. It was also found that modification by lysosome-targeting small molecules could improve the in vitro antitumor activity of ADCs. The schematic diagram is shown in Fig. 27. The present invention provides a simple and versatile method for directing ADCs into lysosomes for effective release of cytotoxic small molecules, and may represent a unique and efficient method for improving the efficacy of ADCs.

Obviously, the above embodiments are merely examples for clarity and are not meant to limit the manner of implementation. For those of ordinary skill in the art, there are other variations or changes that can be made in different forms based on the above description. It is not necessary or possible to exhaust all embodiments here. The obvious variations or changes derived there from are still within the scope of protection of the present invention.

## Claims

1. A lysosome-targeting antibody-drug conjugate, wherein the structure of the antibody-drug conjugate is as follows:
Drₙ₁AbOₙ₂;
wherein, Dr is a drug;
Ab is an antibody;
O is a lysosome-targeting small molecule or a functional peptide for increasing the lysosome-targeting ability of the antibody-drug conjugate;
n1 and n2 are integers greater than or equal to 1, and n1 and n2 are identical or different.

2. The lysosome-targeting antibody-drug conjugate according to claim 1, wherein the antibody is a monoclonal antibody or a nanobody, and the monoclonal antibody is one selected from Glembatumumab, Vandortuzumab, Tisotumab, Enfortumab, Cetuximab, Coltuximab, Lorvotuzumab, Gemtuzumab, Trastuzumab, and Ladiratuzumab; the nanobody is one selected from 7D12, EGA1, 9G8, C7b, 5F7, and 2Rs15d.

3. The lysosome-targeting antibody-drug conjugate according to claim 1, wherein the functional peptide is a lysosome-sorting peptide, a cell-penetrating peptide or a composition of the lysosome-sorting peptide and the cell-penetrating peptide; the functional peptide is conjugated to the C-terminal of the antibody.

4. The lysosome-targeting antibody-drug conjugate according to claim 3, wherein the lysosome-sorting peptide is one selected from NPXY, YXXØ, [DE]XXXL[LI], DXXLL, NPFXD , NPFXXD, wherein the X is any amino acid, the Ø is any amino acid with a bulky hydrophobic side chain, the N is asparagine, the P is proline, the Y is tyrosine, the E is glutamic acid, the L is leucine, the I is isoleucine, the F is phenylalanine, and the D is aspartic acid, the [DE] is represented as one of the D or the E, and the [LI] is represented as one of the L or the I.

5. The lysosome-targeting antibody-drug conjugate according to claim 3, wherein the cell-penetrating peptide is one selected from a cationic cell-penetrating peptide, an amphiphilic cell-penetrating peptide, and a hydrophobic cell-penetrating peptide.

6. The lysosome-targeting antibody-drug conjugate according to claim 5, wherein the cationic penetrating peptide is one selected from polyarginine, HIV-TAT, DPV1047, and Penetratin; the amphiphilic penetrating peptide is one selected from MPG, PVEC, MAP, Pept-1, Transportan, and P28; the hydrophobic penetrating peptide is one selected from C105Y, PFVYLI, and Pep-1.

7. The lysosome-targeting antibody-drug conjugate according to claim 1, wherein the lysosome-targeting small molecule is conjugated to a side chain of an amino acid of the antibody.

8. The lysosome-targeting antibody-drug conjugate according to claim 7, wherein the side chain of the amino acid is a side chain of a natural amino acid or a bioorthogonal group.

9. The lysosome-targeting antibody-drug conjugate according to claim 8, wherein the side chain of the natural amino acid is one selected from a side chain of lysine and a side chain of cysteine; the bioorthogonal group is one selected from an azide group, an alkynyl group, an aldehyde group, a ketone group, and a fluorosulfate group.

10. The lysosome-targeting antibody-drug conjugate according to claim 1, wherein the lysosome-targeting small molecule is a pH-sensitive group-containing compound or a sugar-group containing compound.

11. The lysosome-targeting antibody-drug conjugate according to claim 10, wherein the pH-sensitive group is one selected from sulfonic acid group, 4-morpholinyl group, 2-(2-morpholinoethylamino)ethyl group, and polyethylene glycol group.

12. The lysosome-targeting antibody-drug conjugate according to claim 10, wherein the sugar-group is one selected from glucose, mannose, mannose-6-phosphate, and galactose.

13. Use of the lysosome-targeting antibody-drug conjugate according to any one of claims 1-12 in anticancer drugs.
